# EUROPEAN PATENT APPLICATION

(11) **EP 4 631 504 A1**
(43) Date of publication of application: **15.10.2025**
(21) Application number: 23901146.3
(22) Date of filing: 08.12.2023
(51) Int. Cl.: A61K 31/4409, A61K 31/454, A61K 31/437, A61K 31/517, A61K 38/47, A61P 3/00, A61P 43/00, A61K 31/495, A61K 31/4439

(54) **COMPOSITION FOR PREVENTION OR TREATMENT OF FABRY'S DISEASE**

(30) Priority: 08.12.2022 KR 20220170751; 09.12.2022 KR 20220171568; 28.07.2023 KR 20230098781
(71) Applicant: Korea Advanced Institute of Science and Technology, Daejeon 34141 (KR)
(72) Inventor: HAN, Yong Mahn, Daejeon 34141 (KR); CHOI, Jong Bin, Daejeon 34141 (KR); DO, Hyo Sang, Daejeon 34141 (KR)
(74) Representative: Mewburn Ellis LLP
(86) International application number: PCT/KR2023/020181
(87) International publication number: WO 2024/123129

(57) **Abstract**

The present invention relates to a composition for preventing or treating Fabry disease, and more particularly, to a composition for preventing or treating Fabry disease (FD) comprising a selected FDA-approved, preclinical or clinical compound as an active ingredient, and a composition for preventing or treating Fabry disease, comprising a selected FDA-approved, preclinical or clinical compound and being for use in combination with agalsidase-β (agal).

## Description

### [Technical Field]

The present invention relates to a composition for preventing or treating Fabry disease, and more particularly, to a composition for preventing or treating Fabry disease (FD) comprising a selected FDA-approved, preclinical or clinical compound as an active ingredient, and a composition for preventing or treating Fabry disease, comprising a selected FDA-approved, preclinical or clinical compound and being for use in combination with agalsidase-β (agal).

### [Background Art]

Fabry disease (FD) is an X-linked lysosomal storage disorder which is caused by α-galactosidase (GLA) deficiency. Fabry disease is characterized by the accumulation of globotriaosylceramide (Gb3) in various cell types, particularly vascular endothelial cells. Vascular lesions, including angiokeratomas, usually develop at an early age in FD patients and advance to life-threatening vasculopathies such as left ventricular hypertrophy (LVH), renal failure, and stroke. These complications are assumed to be associated with abnormalities of microvascular function (Rombach SM, Twickler TB, Aerts JMFG, Linthorst GE, Wijburg FA, Hollak CEM (2010) Vasculopathy in patients with Fabry disease: Current controversies and research directions. Molecular Genetics and Metabolism 99: 99-108).

Currently, FD patients are typically treated via enzyme replacement therapy (ERT) using recombinant human agalsidase-β, or via pharmacological chaperone therapy. Fabry disease patients are treated by clearing accumulated Gb3 via enzyme replacement therapy (ERT).

However, ERT has a limited therapeutic effect in patients with late-onset disease or progressive complications. Although ERT can improve the pathophysiologic symptoms of FD temporarily, it is unable to prevent the progression of complications, specifically renal disease, in FD patients. Moreover, the therapeutic effects of ERT are limited because of a short half-life in the body and the high immunogenicity of FD patients. Chaperone therapy is also limited in that it is only effective in FD patients who have particular GLA mutational variations. Therefore, there is a need to develop new therapeutic strategies for vasculopathy in FD.

It has been reported that vascular endothelial cells (VECs) derived from FD patient-derived induced pluripotent stem cells (IDCs) display increased protein levels of thrombospondin-1 (TSP1) and p-SMAD2, as well as hyperactivated SMAD2 signaling, which lead to hyperactivation of the TGF-β signaling pathway, resulting in defective angiogenesis *in vitro* (Do et al., EBioMedicine, 2020) .

In this regard, the inventors of the present application screened for compounds capable of downregulating TSP1 expression and SMAD2 signaling (Korean Patent Application No. 10-2022-0170751).

Meanwhile, TGF-β signaling plays a key role in the endothelial-to-mesenchymal transition (EndMT) of VECs (Cooley et al., 2014), and EndMT results in the development of fibrosis in various tissues. It has been found that, as a hallmark of FD, tissue ischemia due to the accumulation of Gb3 in the microvasculature can result in fibrosis. Although Gb3 accumulation is cleared to some extent by ERT, the therapy is inefficient in FD patients with progressive tissue fibrosis. Therefore, pharmacological manipulation to regulate EndMT would be a useful therapeutic strategy to improve ERT in FD patients.

Fasudil, a clinically approved ROCK signaling inhibitor, reduced the increased levels of TSP1 and p-SMAD2 in FD-VECs and increased the expression of angiogenic factors. Furthermore, fasudil downregulated the endothelial-to-mesenchymal transition (EndMT) and reactive oxygen species production rate of FD-VECs. In addition, oral administration of fasudil to FD animal model mice alleviated several FD phenotypes, including LVH, renal fibrosis, anhidrosis, and heat intolerance. Finally, the inventors of the present application have shown through the above research results that fasudil can be a new therapeutic candidate for FD patients (Choi JB et al., Molecular Therapy, 2023).

Under this technical background, the inventors of the present application have found that the selected inhibitor compound is a potent clinical agent that may be used to rescue impaired vascular function in FD patients and alleviate various symptoms of FD patients, thereby completing the present invention.

### [Disclosure]

### [Technical Problem]

An object of the present invention is to provide a pharmaceutical composition for preventing or treating Fabry disease (FD).

Another object of the present invention is to provide a method for preventing or treating Fabry disease (FD).

Still another object of the present invention is to provide the use of an agent in the manufacture of a medicament for preventing or treating Fabry disease.

Yet another object of the present invention is to provide a composition for preventing or treating Fabry disease by being used in combination with agalsidase-β (agal).

Still yet another of the present invention is to provide a method for preventing or treating Fabry disease (FD) by being used in combination with agalsidase-β (agal).

A further object of the present invention is to provide the use of an agent in the manufacture of a medicament for preventing or treating Fabry disease by being used in combination with agalsidase-β (agal).

### [Technical Solution]

To achieve the above objects, the present invention provides a pharmaceutical composition for preventing or treating Fabry disease (FD), comprising, as an active ingredient, an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor.

The present invention also provides a method for preventing or treating Fabry disease, comprising a step of administering to a subject an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor.

The present invention further provides the use of an agent, selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, in the manufacture of a medicament for preventing or treating Fabry disease.

The present invention also provides a pharmaceutical composition for preventing or treating Fabry disease (FD), comprising, as an active ingredient, an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, and being for use in combination with agalsidase-β (agal).

The present invention also provides a method for preventing or treating Fabry disease, comprising a step of administering to a subject an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor.

The present invention further provides the use of an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor and a ROCK (Rho-associated protein kinase) inhibitor, in combination with agalsidase-beta in the manufacture of a medicament for preventing or treating Fabry disease.

### [Advantageous effects]

According to the present invention, it is possible to provide a novel use of an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor. That is, it is possible to provide the preventive or therapeutic effect of the agent against Fabry disease (FD).

### [Description of Drawings]

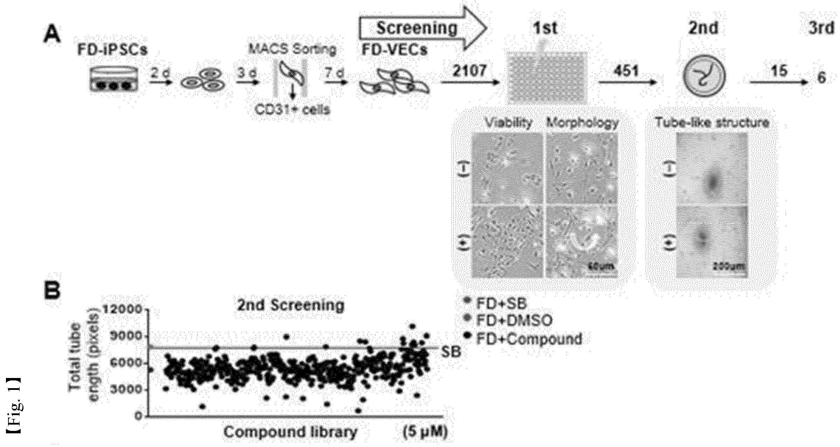

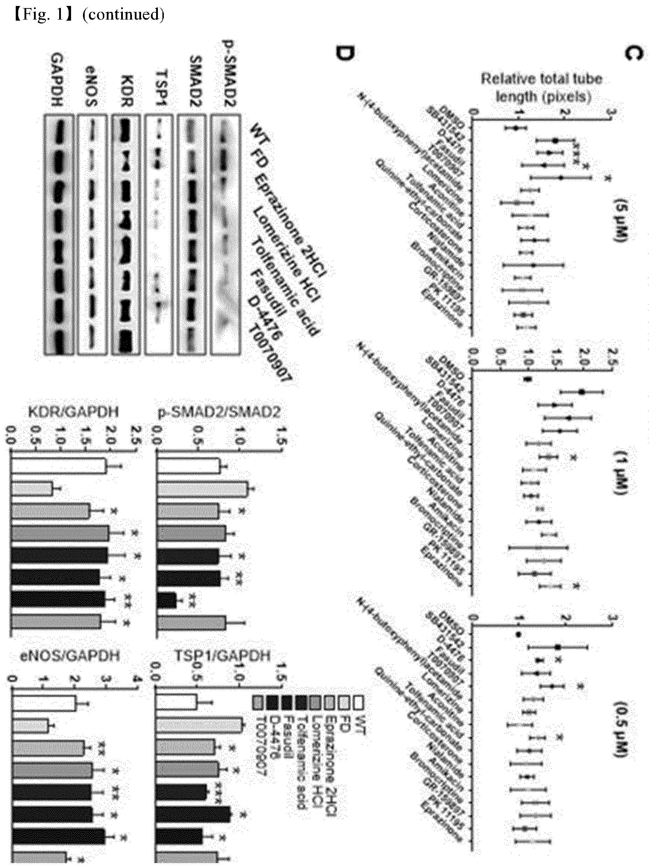

FIG. 1. Screening of hit compounds from a clinical chemical library using FD-VECs.
   (A) Schematic view of the drug screening procedure using FD-VECs that were differentiated from FD-iPSCs. The ability of 2,107 preclinical or clinical chemical compounds to improve the vascular endothelial cell functionality of FD-VECs was examined. In the first screen, compounds inducing cytotoxicity and/or aberrant morphological changes of FD-VECs were excluded. The effects of the compounds on the ability of FD-VECs to form tube-like structures were evaluated in the second and third screens.
   (B) Scatterplot showing the lengths of tubes formed by FD-VECs treated with DMSO (negative control), the TGF-β inhibitor SB431542 (SB; positive control), and the screened clinical compounds (5 µM) in the second screening step.
   (C) The relative total lengths of tubes formed by FD-VECs treated with DMSO (negative control), the TGF-β inhibitor SB431542 (SB; positive control), or the screened clinical compounds (0.5, 1, or 5 µM). Data are presented as mean ± SEM (n = 3). *p < 0.05 and ***p < 0.001 (Student's t test).
   (D) Western blot analysis of p-SMAD2, SMAD2, TSP1, KDR, eNOS, and GAPDH in WT-VECs, untreated FD-VECs, and FD-VECs treated with the screened compounds. Data are presented as mean ± SEM (n = 5). *p < 0.05, **p < 0.01, and ***p < 0.001 (Student's t test).

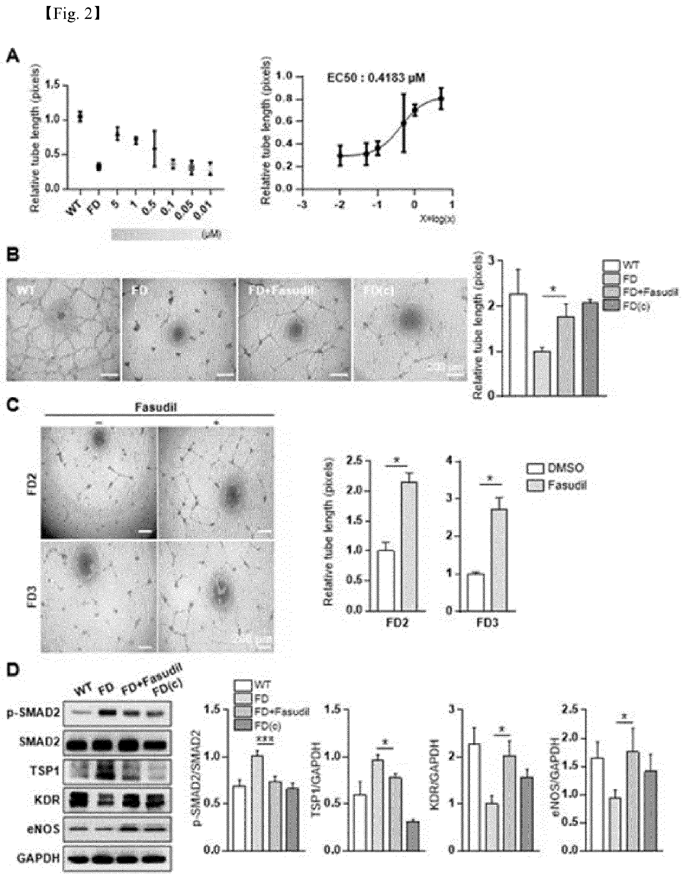

FIG. 2. The effect of fasudil on the angiogenesis of FD-VECs.
   (A) A tube formation assay to determine the optimal concentration and EC50 value of fasudil in FD1-VECs. Data are presented as mean ± SEM (n = 2).
   (B) The effects of fasudil (5 µM) on the tube formation ability of VECs derived from FD1-iPSC lines with distinct GLA mutations. Data are represented as mean ± SEM (n = 3). *p < 0.05 (Student's t-test).
   (C) Analysis of tube-like structure formation by WT-VECs, untreated (FD) or fasudil-treated FD-VECs, and gene-corrected FD-VECs (FD(c)). Data are presented as mean ± SEM (n = 3). p < 0.05 (Student's t test).
   (D) Western blot analysis of p-SMAD2, SMAD2, TSP1, KDR, eNOS, and GAPDH in the cells described in (A). Data are presented as mean ± SEM (n = 7). *p < 0.05 and ***p < 0.001 (Student's t test).
FIG. 3. Fasudil downregulates the EndMT of FD-VECs.
   (A) Western blot analysis of EndMT-associated factors in WT-VECs, untreated (FD) or fasudil-treated FD-VECs, and gene-corrected FD-VECS (FD(c)). Data are presented as mean ± SEM (n = 4). *p < 0.01 (Student's t test).
   (B) Immunostaining of EndMT-associated factors in the cells described in (A).
FIG. 4. Fasudil alleviates impaired metabolic processes in FD-VECs.
   (A) Representative images and analysis showing the fluorescence intensity of ROS in WT-VECs, untreated (FD) or fasudil-treated FD-VECs, and gene-corrected FD-VECs (FD(c)). Data in the graph are presented as mean ± SEM (n = 4). *p < 0.05 and **p < 0.01 (Student's t test).
   (B) Extracellular flux analysis of the oxygen consumption rate (OCR) in the cells described in (A). Data are presented as mean ± SEM (n = 6). O, oligomycin; F, FCCP; R&A, rotenone and antimycin A.

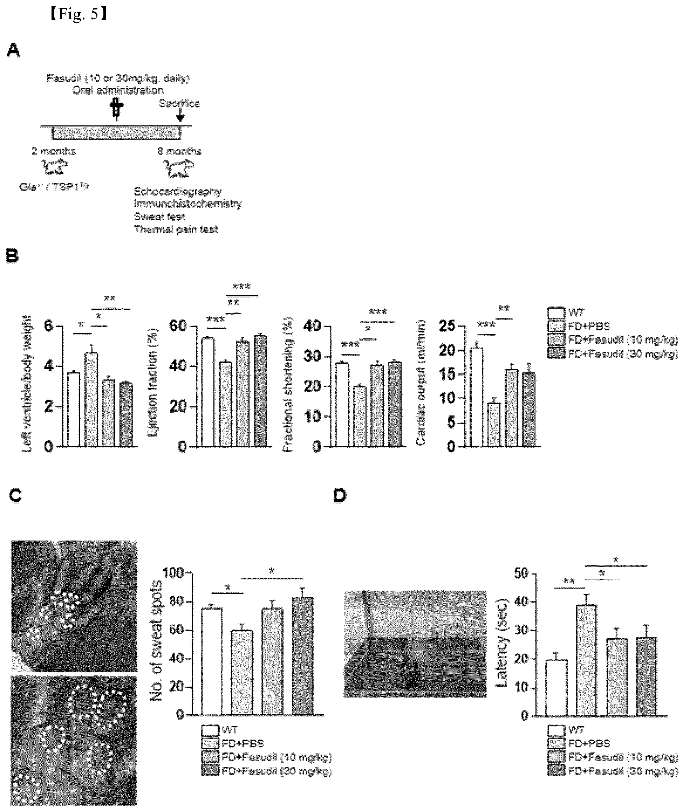

FIG. 5. Oral administration of fasudil rescues FD phenotypes in FD mice.
   (A) Schematic view of an experiment in which fasudil (10 or 30 mg/kg/day for 6 months) is orally administered to FD mice (Gla^{-/-}/TSP1^{Tg}). As a control, FD mice were administered PBS.
   (B) Echocardiography analysis of WT mice (n = 6) and FD mice treated with PBS (n = 4) or fasudil at 10 or 30 mg/kg (n = 9). The cardiac ejection fraction, fractional shortening, and cardiac output were measured. Data are presented as mean ± SEM. *p < 0.05, **p < 0.01, and ***p < 0.001 (Student's t test).
   (C) Analysis of sweat secretion by WT mice and FD mice treated with or without fasudil. Data are presented as mean ± SEM (n = 3). *p < 0.05 (Student's t test).
   (D) Heat tolerance analysis of WT mice and FD mice treated with or without fasudil. Increased latency of paw withdrawal indicates hyposensitivity to thermal pain. Data are presented as mean ± SEM (n = 5). *p < 0.05 (Student's t test).

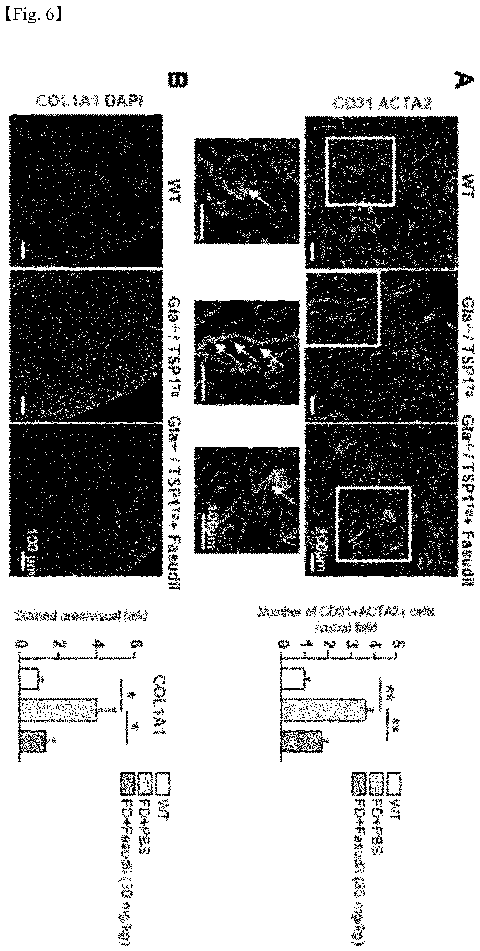

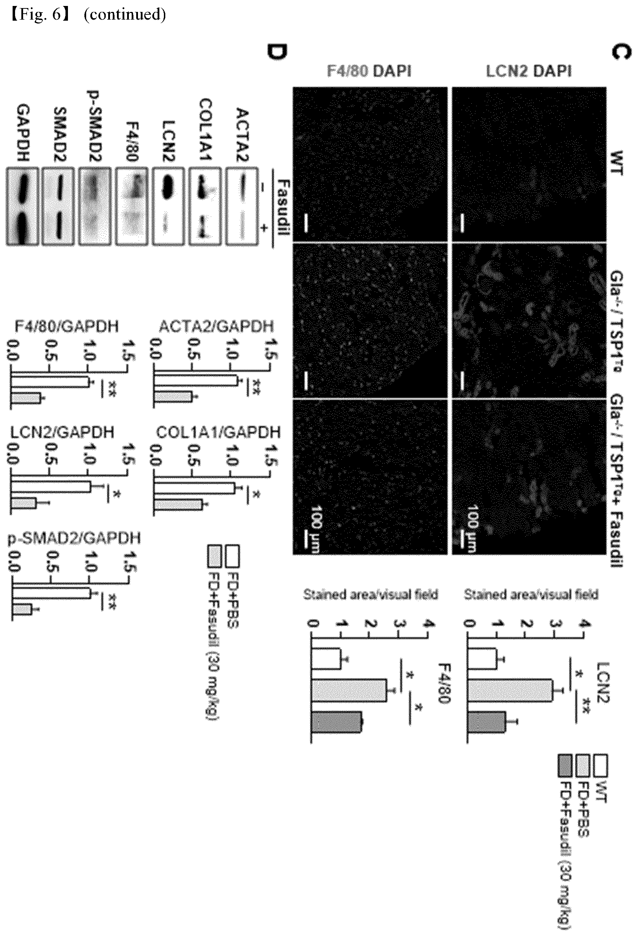

FIG. 6. Oral administration of fasudil alleviates FD phenotypes in Gla^{-/-}/TSP1^{Tg} mice.
   (A) Immunohistochemical analysis of CD31 and ACTA2 in renal tissues from WT mice (n=3) and FD mice (Gla^{-/-}/TSP1^{Tg}) administered PBS (n=3) or fasudil (n=4). Data in the graph are presented as mean ± SEM. ** p < 0.01 (Student's t-test).
   (B) Immunohistochemical analysis of COL1A1 in renal tissues from WT mice (n=3) and FD mice (Gla^{-/-}/TSP1^{Tg}) administered PBS (n=3) or fasudil (n=4). Data in the graph are presented as mean ± SEM. *p < 0.05 (Student's t-test).
   (C) Immunohistochemical analysis of LCN2 and F4/80 in renal tissues from WT mice (n=3) and FD mice (Gla^{-/-}/TSP1^{Tg}) administered PBS (n=3) or fasudil (n=4). Data in the graph are presented as mean ± SEM. *p < 0.05 and **p < 0.01 (Student's t-test).
   (D) Western blot analysis of fibrosis-related markers (ACTA2 and COL1A1), inflammation markers (LCN2 and F4/80), p-SMAD2, SMAD2, and GAPDH in renal tissues from fasudil-treated FD mice (Gla^{-/-}/TSP1^{Tg}). Data are presented as mean ± SEM (n = 3). *p < 0.05 and **p < 0.01 (Student's t-test).

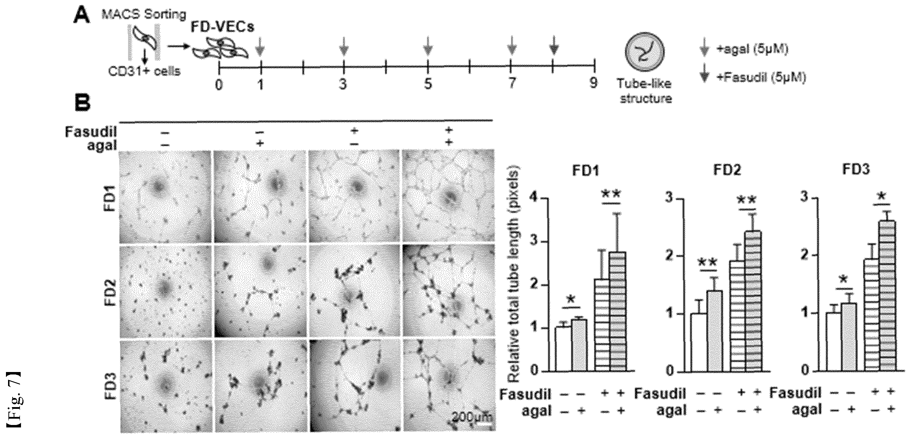

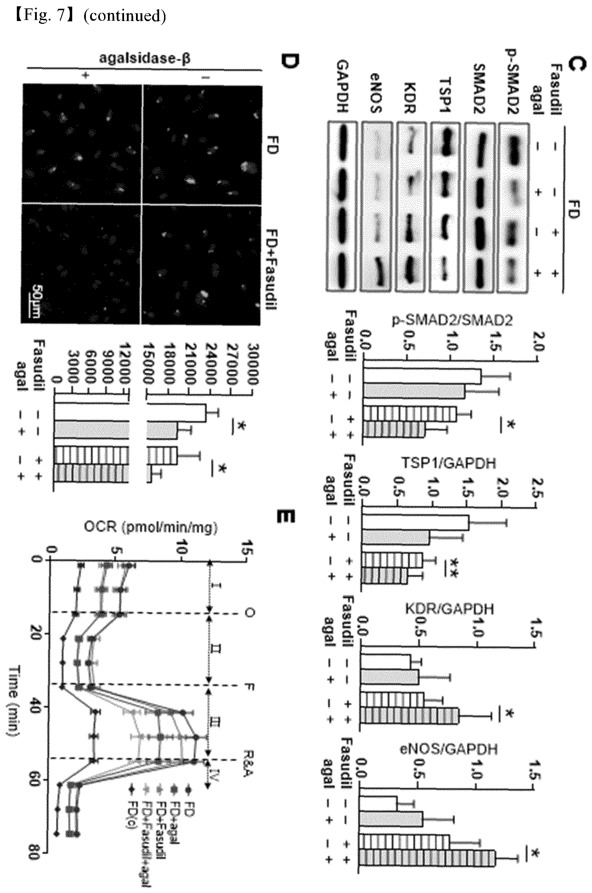

FIG. 7. Synergistic effect of treatment with a combination of fasudil and recombinant human agalsidase-β on the function of FD-VECs.
   (A) Schematic view of the experiment for co-treatment of FD-VECs differentiated from FD-iPSCs with fasudil and agal. FD-VECs were treated with Aal every 2 days, and then treated once with fasudil on day 8. The following day, the tube formation ability of FD-VECs was evaluated.
   (B) Tube formation assay in VECs derived from FD-iPSCs with distinct GLA mutations under co-treatment with fasudil and/or agal. Data in the graph are presented as mean ± SEM (n=3). *p < 0.05 and **p < 0.01 (Student's t-test).
   (C) Western blot analysis of SMAD2, TSP1, KDR, eNOS, and GAPDH in the cells described in (B). Data in the graph are presented as mean ± SEM (n=7). *p < 0.05 and **p < 0.01 (Student's t-test).
   (D) Representative images and analysis of the fluorescence intensity of ROS in the cells described in (B). Data in the graph are presented as mean ± SEM (n = 5). *p < 0.05 and **p < 0.01 (Student's t-test). Scale bar: 50 µm.
   (E) Extracellular flux analysis of the oxygen consumption rate (OCR) in the cells described in (B). Data are presented as mean ± SEM (n = 6). O, oligomycin; F, FCCP; R&A, rotenone & antimycin A.

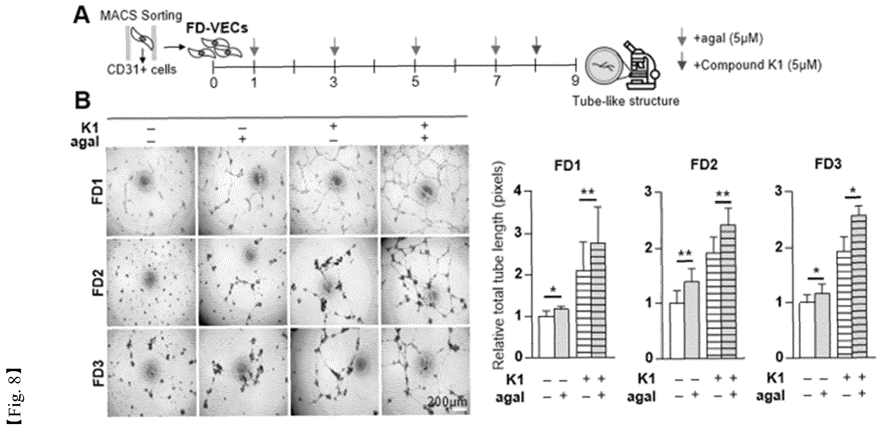

FIG. 8 shows the synergistic effect of co-treatment with fasudil and recombinant human agalsidase-β on the function of FD vascular endothelial cells.

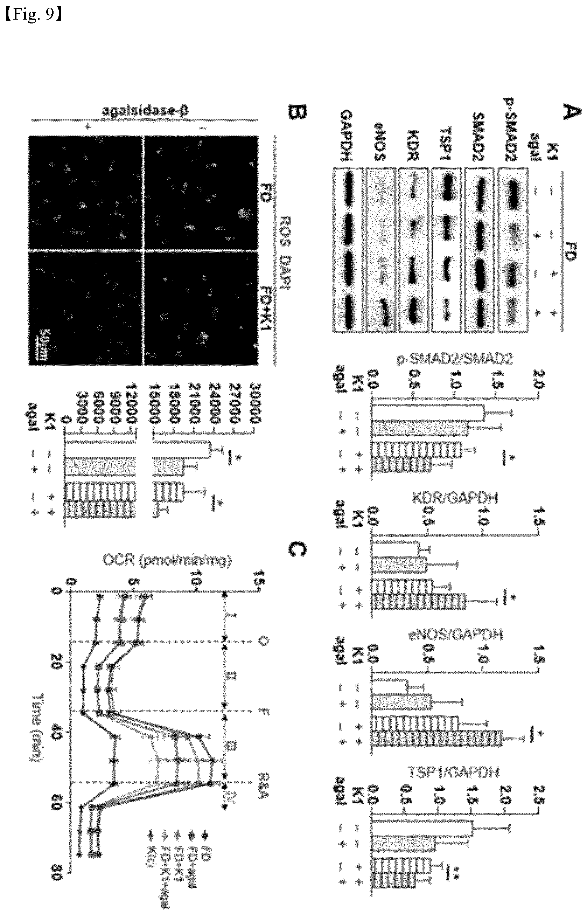

FIG. 9 shows the synergistic effect of co-treatment with fasudil and recombinant human agalsidase-β on the enhancement of metabolic function in FD vascular endothelial cells.

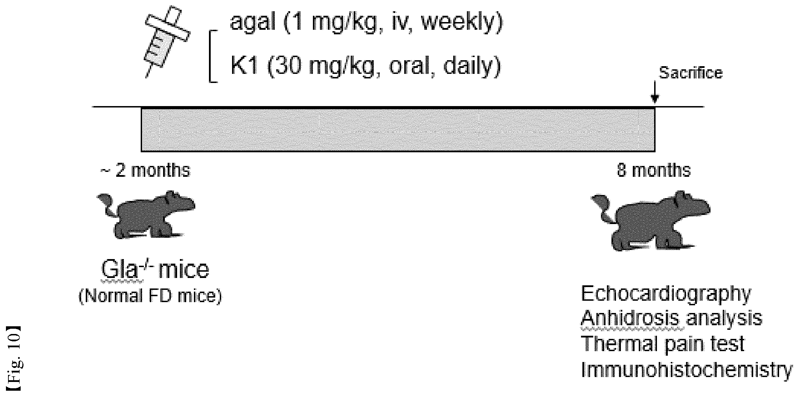

FIG. 10 shows a method of co-administering fasudil and recombinant human agalsidase-β to Gla^{-/-} model mice.

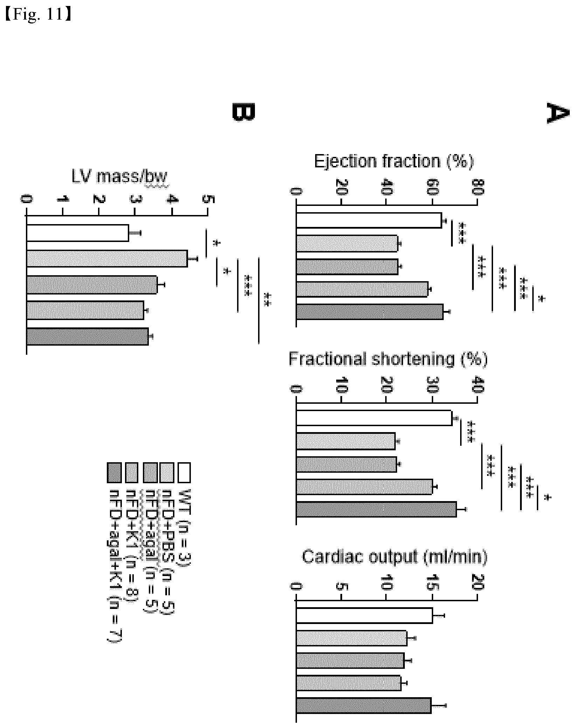

FIG. 11 shows the results of echocardiography analysis of mice with Fabry disease after co-administration of fasudil and recombinant human agalsidase-β.

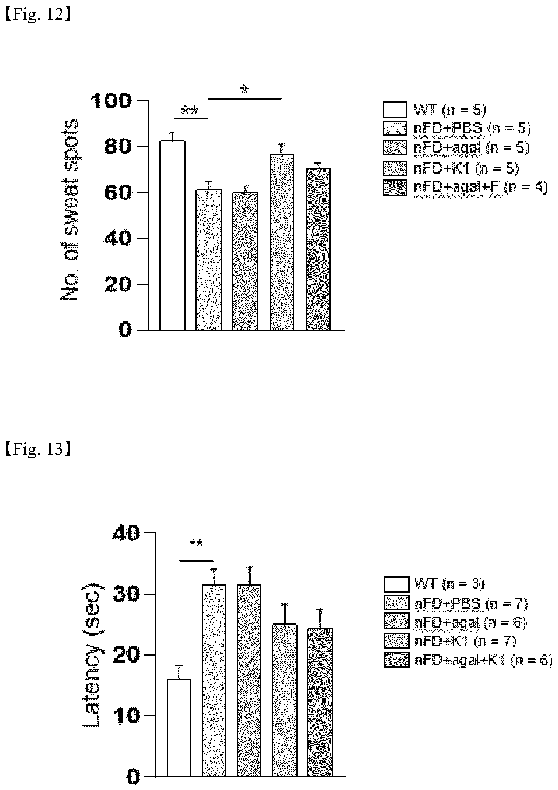

FIG. 12 shows the results of analysis of sweat secretion by mice with Fabry disease after co-administration of fasudil and recombinant human agalsidase-β.
FIG. 13 shows the results of analysis of peripheral nerves of mice with Fabry disease after co-administration of fasudil and recombinant human agalsidase-β.

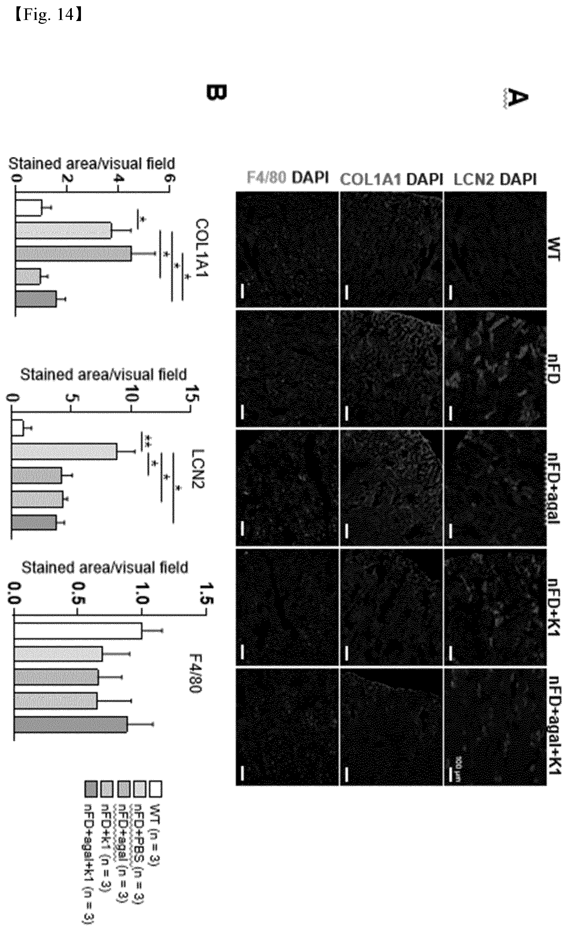

FIG. 14 shows the results of analysis of the renal tissues of mice with Fabry disease after co-administration of fasudil and recombinant human agalsidase-β.

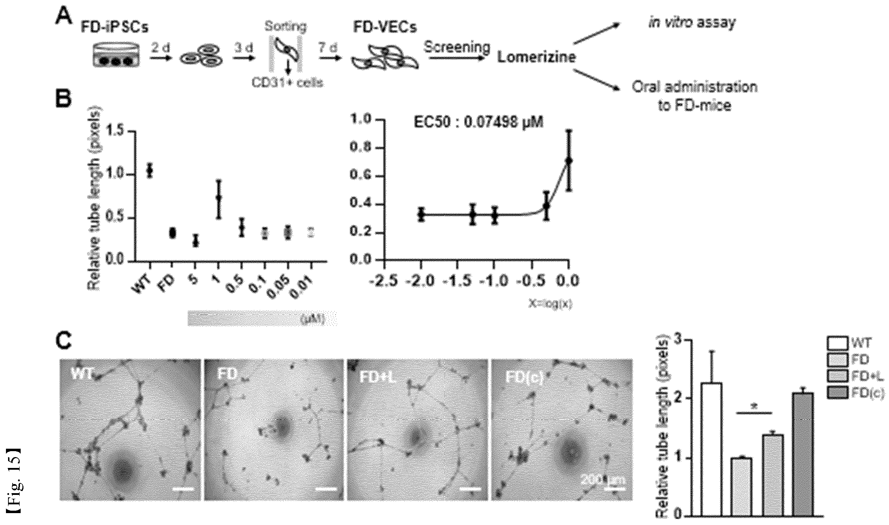

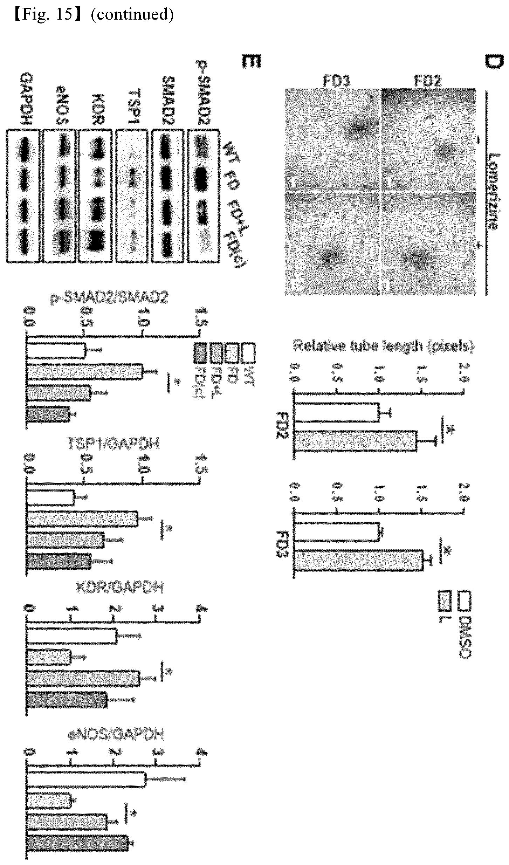

FIG. 15. Lomerizine improves impaired angiogenesis of FD-VECs.
   (A) Schematic view of the drug screening procedure using FD-VECs. FD-VECs were obtained by differentiation from FD-iPSCs for phenotypic drug screening. Based on FDA-approved clinical compounds, drugs with the ability to improve the vascular endothelial cell function of FD-VECs were identified. In the first screening step, compounds inducing cytotoxicity or aberrant morphological changes were excluded. In the next step, the remaining compounds were screened for their ability to affect the tube formation ability of FD-VECs. Finally, the therapeutic effect of the most effective compound was tested in FD-mice.
   (B) The results of observing the tube formation ability at various concentrations and analyzing the EC50 based on the data, in order find out how much lomerizine rescues the tube formation ability of FD-VECs.
   (C) Tube-like structure formation by WT-VECs, untreated FD-VECs (FD), lomerizine-treated FD-VECs, and gene-corrected FD-VECs (FD(c)). Lomerizine-treated FD-VEC showed significantly increased tube formation ability compared to the untreated group. Data are presented as mean ± SEM (n = 3). p < 0.05 (Student's t-test); Scale bar: 200 µm; WT, wild type, normal; FD, Fabry disease; L, lomerizine.
   (D) Results showing that lomerizine treatment can improve the tube formation ability of FD-VECs differentiated from patient iPSCs with different GLA mutations.
   (E) Western blot analysis for the cells described in (C). Treatment of FD-VECs with lomerizine reduced the levels of p-SMAD2 and TSP1 and increased the levels of KDR and eNOS. Data are presented as mean ± SEM (n = 4). *p < 0.05 and ***p < 0.001 (Student's t-test).

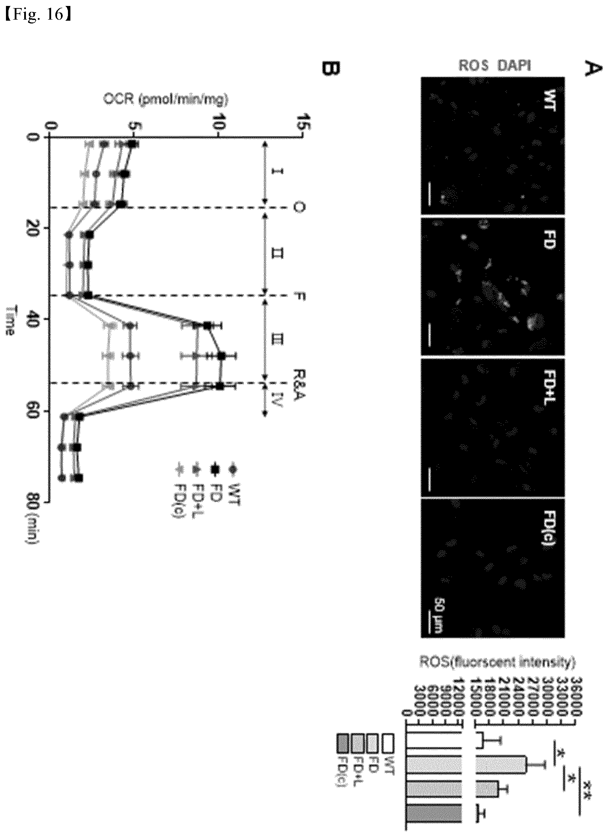

FIG. 16. Lomerizine alleviates the mitochondrial dysfunction of FD-VECs.
   (A) Representative images and analysis of ROS fluorescence intensity in WT-VECs, untreated FD-VECs (FD), lomerizine-treated FD-VECs, and gene-corrected FD-VEC (FD(c)). Lomerizine reduced ROS generation in FD-VECs. Data in the graph are presented as mean ± SEM (n = 4). *p < 0.05 and **p < 0.01 (Student's t-test).
   (B) Extracellular flux analysis of oxygen the consumption rate (OCR) in the cells described in (A). Lomerizine reduced maximal respiration in FD-VECs. Data are presented as mean ± SEM (n = 6). O, oligomycin; F, FCCP; R&A, rotenone and antimycin A.

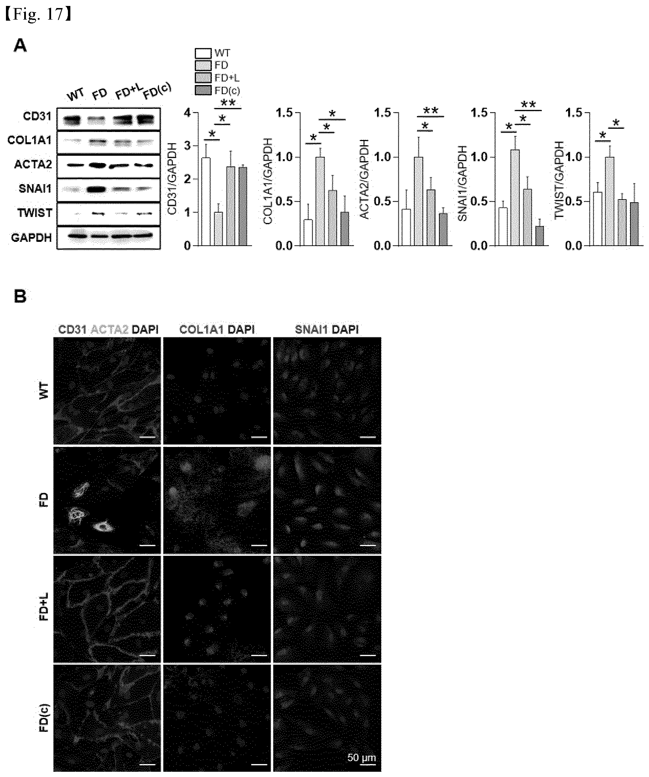

FIG. 17. Lomerizine improves FD-VEC function by downregulating EndMT.
   (A) Western blot for the EndMT-related factors CD31, COL1A1, ACTA2, SNAI1, and TWIST in WT-VECs, untreated FD-VECs (FD), lomerizine-treated FD-VECs, and gene-corrected FD-VECs (FD(c)). Lomerizine increased CD31 levels and decreased EndMT-related proteins in FD-VECs. Data are presented as mean ± SEM (n = 4). *p < 0.05 (Student's t-test).
   (B) Immunostaining of EndMT-related proteins in the cells described in (A). Lomerizine reduced the levels of EndMT-related proteins in FD-VECs. Scale bar: 50 µm.

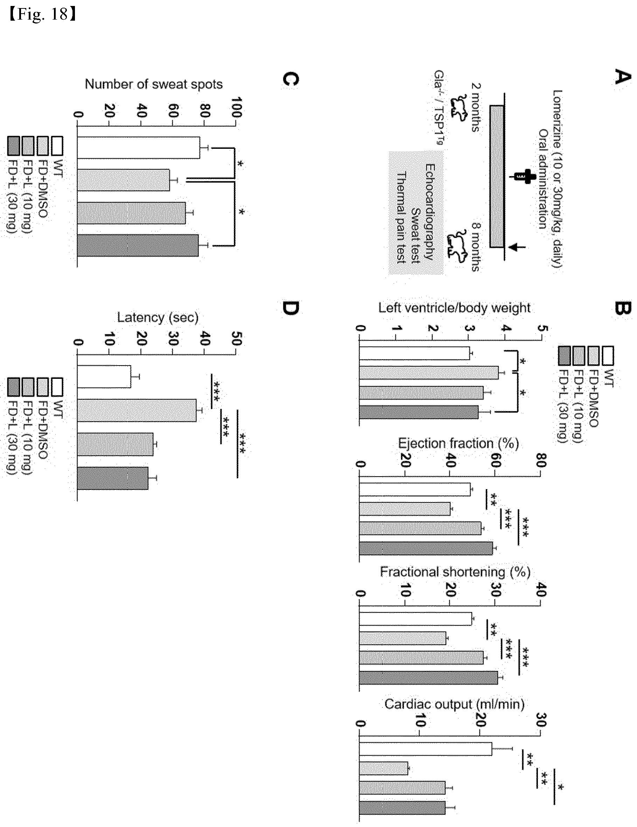

FIG. 18. Oral administration of lomerizine rescues the FD phenotypes of FD mice.
   (A) Schematic outline of the protocol by which FD-mice (Gla^{-/-}/TSP1^{Tg}) were treated with lomerizine (10 or 30 mg/kg/day for 6 months). Next, the mice were subjected to echocardiography, sweat secretion tests, and thermal pain tests. Control FD-mice received oral DMSO.
   (B) Echocardiographic measurements of cardiac function in WT (n=3) and FD-mice treated with DMSO (n=4) or lomerizine at 10 or 30 mg/kg (n= 9). The left ventricle mass/body weight, ejection fraction, fractional shortening, and cardiac output were measured. Lomerizine improved the cardiac function of FD-mice. Data are presented as means ± SEM. *p< 0.05, **p<0.01, and ***p<0.001 (Student's t-test).
   (C) Analysis of sweat secretion from WT mice (n=3) and FD mice treated with or without lomerizine. Data are presented as means ± SEM (n=3). *p<0.05 and **p<0.01 (Student's t-test).
   (D) Heat tolerance analysis of WT mice (n=3) and FD mice treated with or without lomerizine (n=5). Increased latency of paw withdrawal indicates hyposensitivity to thermal pain. Data are presented as means ± SEM. *p<0.05 and **p < 0.01 (Student's t-test).

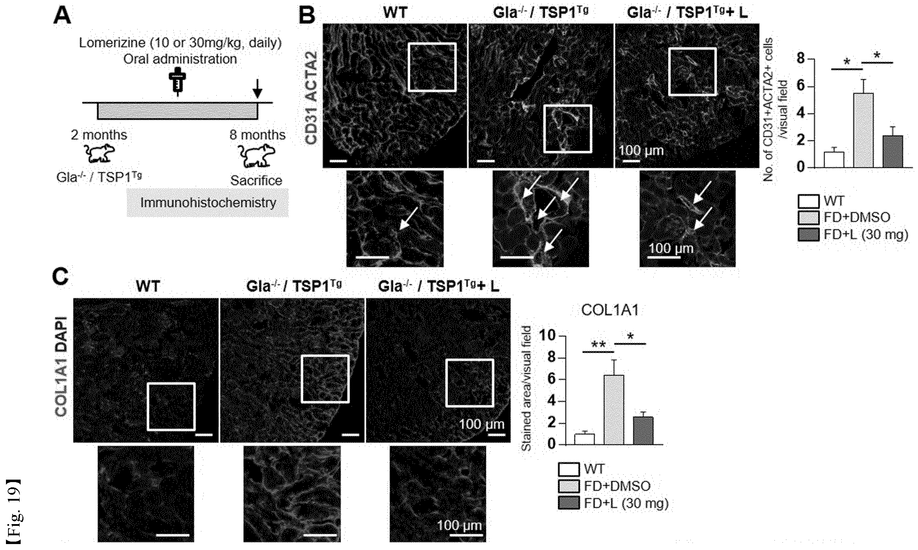

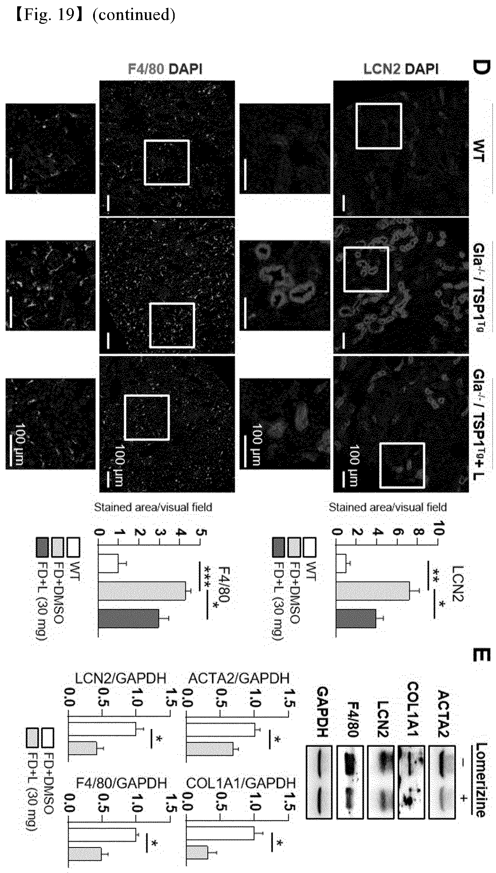

FIG. 19. Oral administration of lomerizine alleviates fibrosis and inflammation in the renal tissues of FD-mice.
   (A) Schematic outlining of the protocol by which FD-mice (Gla^{-/-}/TSP1^{Tg}) were treated with lomerizine (10 or 30 mg/kg/day for 6 months) prior to the analysis of their renal tissues. Control FD-mice received oral DMSO.
   (B) Immunohistochemistry of CD31 and ACTA2 in renal tissues from WT mice (n = 3) and FD-mice (Gla^{-/-}/TSP1^{Tg}) administered DMSO (n=3) or lomerizine (n=4). Lomerizine reduced ACTA2 fluorescence from CD31+cells in the renal tissues of FD-mice. Data in the graph are presented as means ± SEM. *p<0.05 (Student's t-test).
   (C) Immunohistochemistry of COL1A1 in renal tissues from WT (n=3) and FD-mice (Gla^{-/-}/TSP1^{Tg}) administered DMSO (n=3) or lomerizine (n=4). Lomerizine reduced COL1A1 fluorescence from the renal tissues of FD-mice. Data in the graph are presented as means±SEM. *p<0.05 and **p<0.01 (Student's t-test).
   (D) Immunohistochemistry of the inflammation-associated markers F4/80 and LCN2 in renal tissues from WT (n=3) and FD-mice (Gla^{-/-}/TSP1^{Tg}) administered DMSO (n=3) or lomerizine (n=4). Lomerizine reduced F4/80 and LCN2 florescence from the renal tissues of FD-mice. Data in the graph are presented as means±SEM; *p<0.05, **p<0.01, and ***p<0.001 (Student's t-test).
   (E) Western blot analyses of the fibrosis-related markers ACTA2 and COL1A1, the inflammation markers LCN2 and F4/80, as well as GAPDH in kidney lysates from FD-mice. The expression of these fibrosis- and inflammation-related markers was reduced in FD mice treated with lomerizine. Data are presented as means±SEM (n=3). *p<0.05 (Student's t-test) .

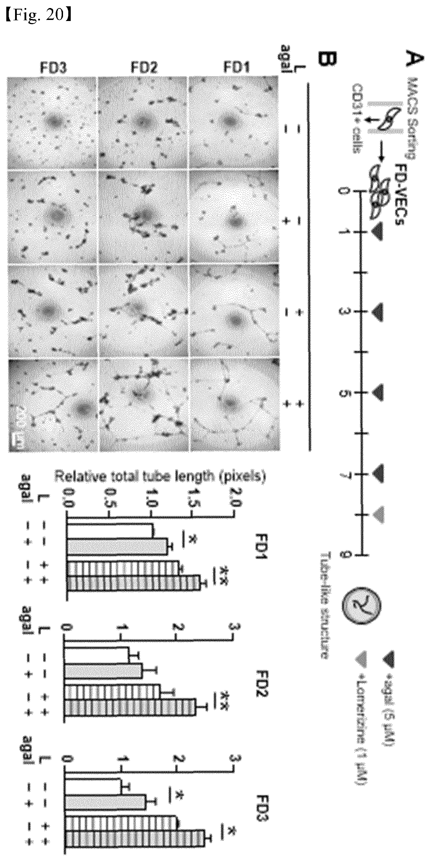

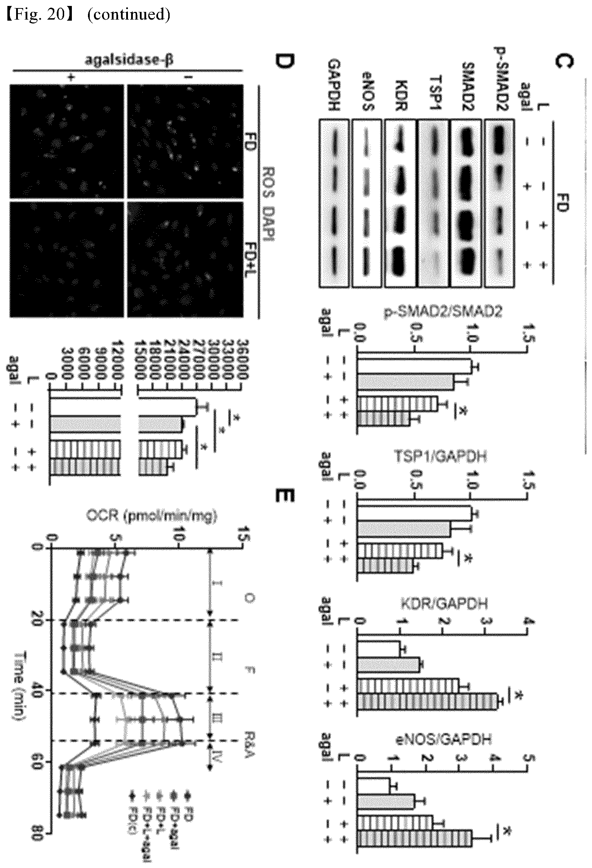

FIG. 20. Lomerizine exhibits a therapeutic synergistic effect on the function of FD-VECs when used in combination with recombinant human agalsidase-β.
   (A) Schematic diagram of the experiment for co-treatment of FD-VECs differentiated from FD-iPSCs with lomerizine and agal. FD-VECs were treated with agal every 2 days, and then treated once with fasudil on day 8. The following day, the tube formation ability of FD-VECs was evaluated.
   (B) Tube formation assay in VECs derived from FD-iPSCs with different GLA mutations under co-treatment with lomerizine and/or agal. Data in the graph are presented as mean ± SEM (n=3). *p < 0.05 and **p < 0.01 (Student's t-test).
   (C) Western blot analysis of SMAD2, TSP1, KDR, eNOS, and GAPDH in the cells described in (B). Data in the graph are presented as mean ± SEM (n=5). *p < 0.05 and **p < 0.01 (Student's t-test).
   (D) Representative images and analysis of the fluorescence intensity in the cells described in (B). Data in the graph are presented as mean ± SEM (n = 5). *p < 0.05 and **p < 0.01 (Student's t-test). Scale bar: 50 µm.
   (E) Extracellular flux analysis of the oxygen consumption rate (OCR) in the cells described in (B). Data are presented as mean ± SEM (n = 6). O, oligomycin; F, FCCP; R&A, rotenone & antimycin A.

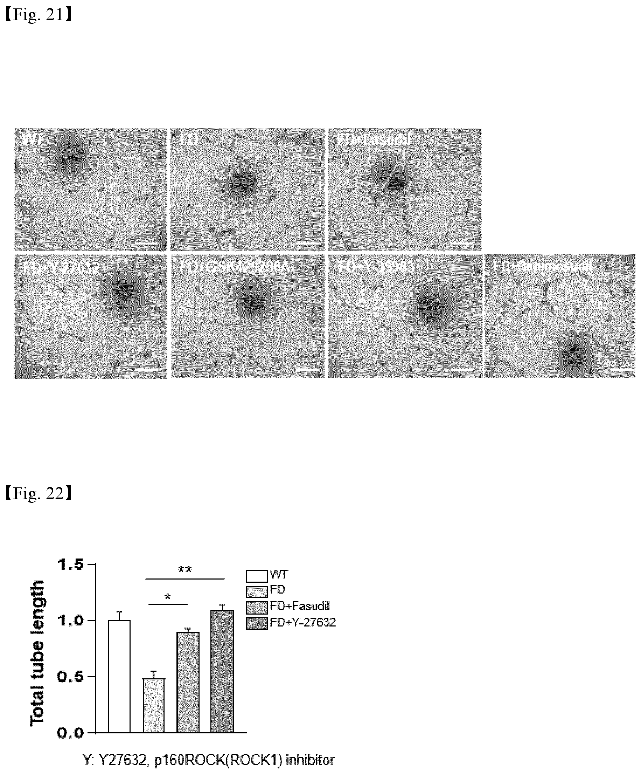

FIG. 21. Images showing the analysis of tube formation of Fabry disease vascular endothelial cells by treatment with various ROCK inhibitors.
FIG. 22 shows the effect of ROCK inhibitor (Y-27632) treatment on increasing the tube formation ability of Fabry disease vascular endothelial cells.

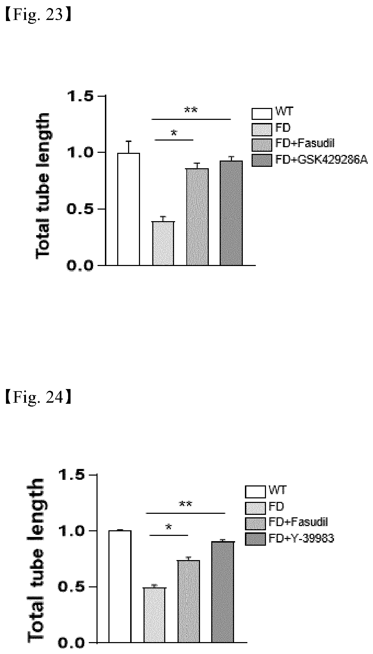

FIG. 23 shows the effect of ROCK inhibitor (GSK429286A) treatment on increasing the tube formation ability of Fabry disease vascular endothelial cells.
FIG. 24 shows the effect of ROCK inhibitor (Y-39983) treatment on increasing the tube formation ability of Fabry disease vascular endothelial cells.

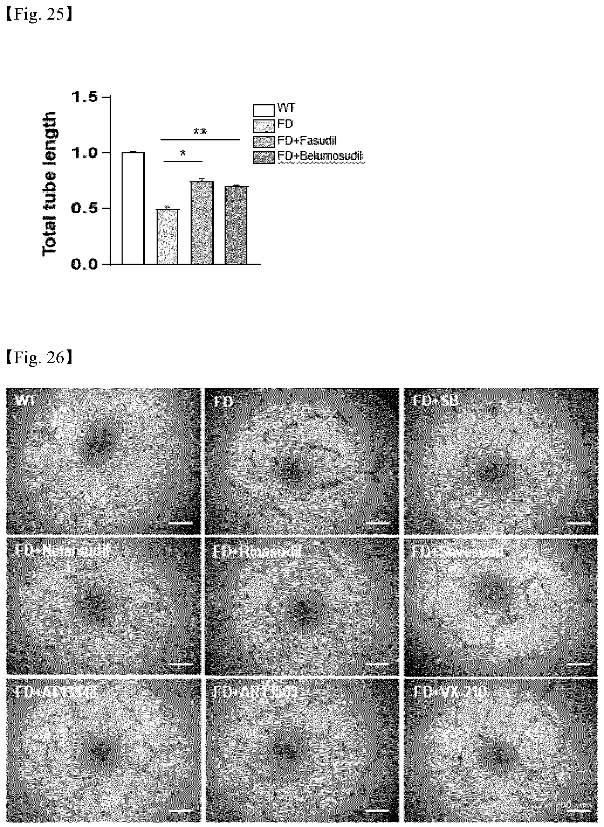

FIG. 25 shows the effect of ROCK inhibitor (belumosudil) treatment on increasing the tube formation ability of Fabry disease vascular endothelial cells.
FIG. 26. Images showing the analysis of tube formation of Fabry disease endothelial cells by treatment with various ROCK inhibitors. WT, wild type; FD, Fabry disease, SB: SB431542, a SMAD2 signaling inhibitor.

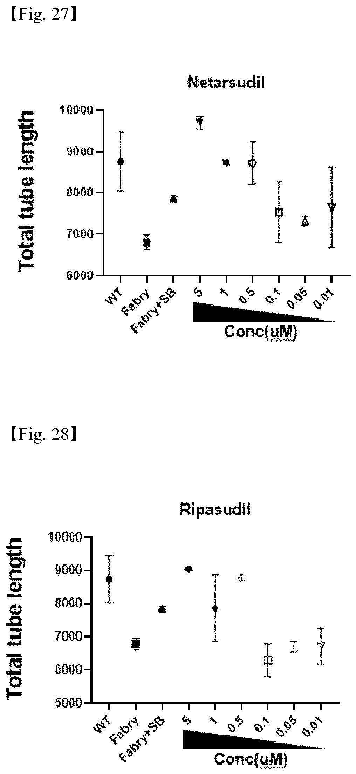

FIG. 27 shows the effect of ROCK inhibitor (netarsudil) treatment on increasing the tube formation ability of Fabry disease vascular endothelial cells.
FIG. 28 shows the effect of ROCK inhibitor (ripasudil) treatment on increasing the tube formation ability of Fabry disease vascular endothelial cells.

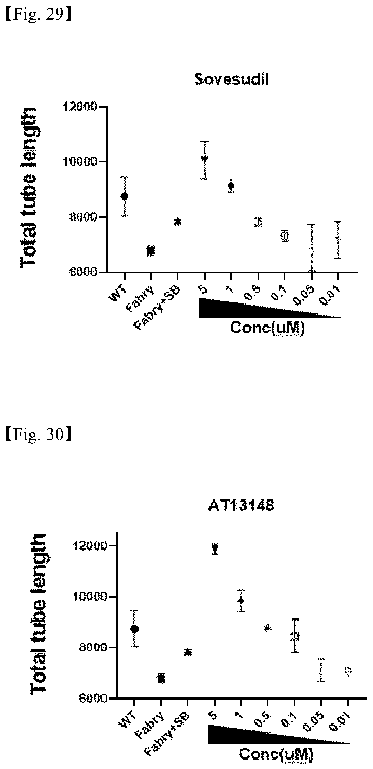

FIG. 29 shows the effect of ROCK inhibitor (sovesudil) treatment on increasing the tube formation ability of Fabry disease vascular endothelial cells.
FIG. 30 shows the effect of ROCK inhibitor (AT13148) treatment on increasing the tube formation ability of Fabry disease vascular endothelial cells.

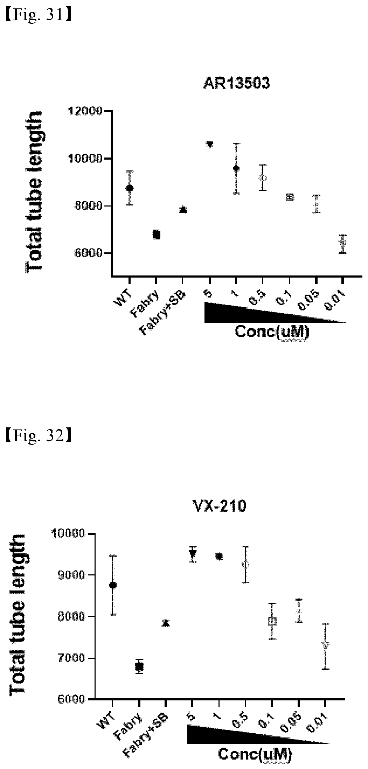

FIG. 31 shows the effect of ROCK inhibitor (AR13503) treatment on increasing the tube formation ability of Fabry disease vascular endothelial cells.
FIG. 32 shows the effect of ROCK inhibitor (VX-210) treatment on increasing the tube formation ability of Fabry disease vascular endothelial cells.

### [Mode for Invention]

Unless otherwise defined, all technical and scientific terms used in the present specification have the same meanings as commonly understood by those skilled in the art to which the present disclosure pertains. In general, the nomenclature used in the present specification is well known and commonly used in the art.

The inventors of the present application screened a library of 2,107 FDA-approved, preclinical and clinical compounds to identify drugs that may be useful therapeutic agents for Fabry disease (FD).

There among, an agent selected from the group consisting of transforming growth factor-β (TGF-β) receptor activator inhibitors, peroxisome proliferator-activated receptor (PPARγ) antagonists, calcium channel blockers, cyclooxygenase (COX) inhibitors, piperazine-based compounds, and Rho kinase inhibitors reduced the protein levels of p-SMAD2 and TSP1 in FD-VECs, and increased the levels of the angiogenic factors KDR and eNOS. In addition, fasudil inactivated TGF-β-induced EndMT and reduced reactive oxidative stress (ROS) and maximal respiration in FD-VECs. Furthermore, *in vivo,* oral administration of fasudil to transgenic mice expressing human TSP1 (Gla^{-/-}/TSP1^{Tg}; FD mice]) rescued several FD phenotypes, including LVH, renal fibrosis, anhidrosis, and heat intolerance.

There among these, calcium channel blockers reduced the protein levels of p-SMAD2 and TSP1 in FD-VECs and increased the levels of the angiogenic factors KDR and eNOS. In addition, calcium channel blockers effectively inhibited EndMT of FD-VECs by downregulating ROS generation and reducing maximal mitochondrial respiration. It was found that oral administration to FD mice (Gla^{-/-}/TSP1^{Tg}) alleviated several FD phenotypes, including LVH, renal fibrosis, anhidrosis, and heat intolerance.

In addition, in the present invention, it was examined whether 10 types of ROCK signaling inhibitors with a functionality similar to that of fasudil could rescue the endothelial dysfunction of FD-VECs, and as a result, it was confirmed that these inhibitors improved the endothelial functionality of FD-VECs. Thereby, the inventors of the present application have shown that ROCK signaling inhibitors may be helpful in the treatment of Fabry disease patients.

The inventors of the present application suggest that fasudil is a potent clinical compound that may be used to rescue dysfunctional angiogenesis and improve several symptoms in FD patients.

The inventors of the present application suggest that a calcium channel blocker is a potent clinical compound that may be used to rescue dysfunctional angiogenesis and improve several symptoms in FD patients.

In addition, the inventors of the present application screened ROCK (Rho-associated protein kinase) inhibitors to identify drugs that could be useful therapeutic agents for Fabry disease (FD) and confirmed that the screened compounds exhibited an effect of improving the tube formation ability of Fabry disease vascular endothelial cells.

Based on these findings, the present invention relates to a pharmaceutical composition for preventing or treating Fabry disease (FD), comprising, as an active ingredient, an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor.

Fabry disease is an X-linked recessive genetic disease resulting from mutations in the gene encoding alpha-galactosidase (GLA). The GLA gene is a gene located at xq22.1 of exon 7 in humans and encodes a glycoprotein consisting of 370 amino acids processed from a precursor protein consisting of a total of 429 amino acids.

More than 400 mutation sites known to appear in the GLA gene have been reported (http://www.hgmd.cf.ac.uk), and the severity of Fabry disease symptoms varies depending on the mutation location in the GLA gene. The activity of alpha-galactosidase is not exhibited at all by most of the mutations, but residual enzyme activity of 5 to 10% is exhibited by some missense mutations. Patients with these mutations do not display clinically important pathophysiology.

The major pathophysiological symptom of Fabry disease is the accumulation of Gb3 in various cell types, such as vascular cells, cardiac cells, kidney epithelial cells, and neuronal cells.

Agalsidase-β, an alpha-galactosidase, is administered to clear accumulated Gb3 from various cell types. The enzyme administered by intravenous injection enters the cell through the mannose 6-phosphate (M6P) receptor on the plasma membrane and moves to the lysosome.

The TGF-β receptor activity inhibitor may be SB431542 or D-4476 comprising the following structure or a pharmaceutically acceptable salt thereof: 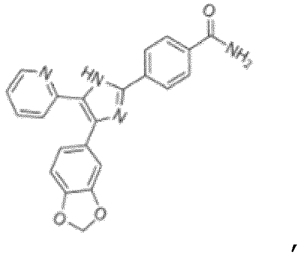 or

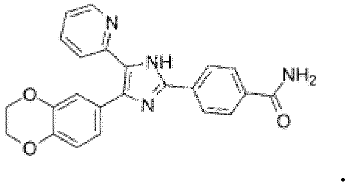

The PPARγ antagonist may be T0070907 comprising the following structure or a pharmaceutically acceptable salt thereof:

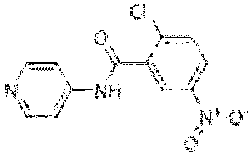

The calcium channel blocker may be lomerizine comprising the following structure or a pharmaceutically acceptable salt thereof:

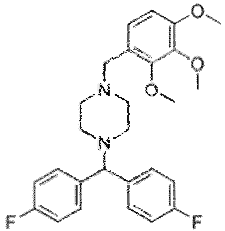

The COX inhibitor may be tolfenamic acid comprising the following structure or a pharmaceutically acceptable salt thereof:

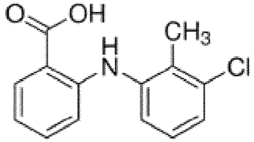

The piperazine-based compound may be eprazinone or a pharmaceutically acceptable salt thereof:

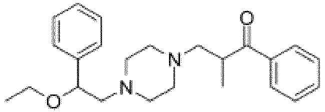

The Rho kinase inhibitor may be fasudil comprising the following structure or a pharmaceutically acceptable salt thereof:

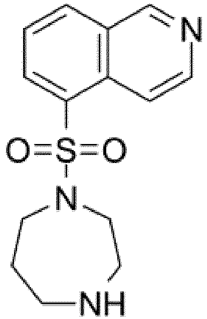

The ROCK inhibitor may comprise at least one selected from the group consisting of Y27632, GSK429286A, Y39983, belumosudil, netarsudil mesylate, ripasudil hydrochloride hydrate, AR-13503 (main netarsudil metabolite), AMA-0076 (sovesudil), AT-13148, and VX-210, or a pharmaceutically acceptable salt thereof.

The "pharmaceutically acceptable salt" may be an acid addition salt formed by a pharmaceutically acceptable free acid, wherein the free acid may be an organic acid or an inorganic acid.

Examples of the organic acids include, but are not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid, and aspartic acid. In addition, examples of the inorganic acid include, but are not limited to, hydrochloric acid, hydrobromic acid, sulfuric acid, and phosphoric acid.

For example, if the compound is anionic, or has a functional group which may be anionic (e.g., -COOH may be - COO⁻), then a salt may be formed with a suitable cation. Examples of suitable inorganic cations include, but are not limited to, alkali metal ions such as Na⁺ and K⁺, alkaline earth metal cations such as Ca²⁺ and Mg²⁺, and other cations such as Al³⁺. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺).

Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)⁴⁺.

If the compound is cationic or has a functional group that may be cationic (e.g., -NH₂ may be -NH₃ ⁺), then a salt may be formed with a suitable anion. Examples of suitable inorganic anions include, but are not limited to, those derived from the following inorganic acids: hydrochloric acid, hydrobromic acid, hydroiodic acid, sulfuric acid, sulfurous acid, nitric acid, nitrous acid, phosphoric acid, and phosphorous acid.

Examples of suitable organic anions include, but are not limited to, those derived from the following organic acids: 2-acetyoxybenzoic acid, acetic acid, ascorbic acid, aspartic acid, benzoic acid, camphorsulfonic acid, cinnamic acid, citric acid, edetic acid, ethanedisulfonic acid, ethanesulfonic acid, fumaric acid, glucoheptonic acid, gluconic acid, glutamic acid, glycolic acid, hydroxymaleic acid, hydroxynaphthalene carboxylic acid, isethionic acid, lactic acid, lactobionic acid, lauric acid, maleic acid, malic acid, methanesulfonic acid, mucic acid, oleic acid, oxalic acid, palmitic acid, pamoic acid, pantothenic acid, phenylacetic acid, phenylsulfonic acid, propionic acid, pyruvic acid, salicylic acid, stearic acid, succinic acid, sulfanilic acid, tartaric acid, toluenesulfonic acid, and valeric acid. Examples of suitable polymeric organic anions include, but are not limited to, those derived from the following polymeric acids: tannic acid, carboxymethyl cellulose, and the like.

Specifically, the Rho kinase inhibitor may comprise fasudil hydrochloride.

The pharmaceutical composition according to the present invention may be administered by any route. The composition of the present invention may be provided to an animal by any suitable means, directly (e.g., locally, as by injection, implantation or topical administration to a tissue locus) or systemically (e.g., parenterally or orally). Where the composition of the present invention is to be provided parenterally, such as by intravenous, subcutaneous, ophthalmic, intraperitoneal, intramuscular, buccal, rectal, vaginal, intraorbital, intracerebral, intracranial, intraspinal, intraventricular, intrathecal, intracisternal, intracapsular, intranasal or by aerosol administration, the pharmaceutical composition may comprise, for example, part of an aqueous or physiologically compatible fluid suspension or solution. Accordingly, the carrier or vehicle is physiologically compatible, and thus it may be added to the composition and delivered to the patient. Therefore, a physiologically-appropriate saline solution may generally be included as a carrier such as a fluid medium for formulation.

Further, the frequency of administration may vary depending on the pharmacokinetic parameters of the formulations used. Typically, physicians will administer the formulation until a dosage is reached that achieves the desired effect. Accordingly, the pharmaceutical composition may be administered as a single dose, or as two or more doses over time, or as a continuous infusion via an implantation device or catheter. Further refinement of the appropriate dosage is routinely made by those of ordinary skill in the art and is within the ambit of tasks routinely performed by them.

In addition, the unit dosage in humans is 0.01 µg/kg to 100 mg/kg, specifically, 1 µg/kg to 10 mg/kg of body weight. Although the above content is the optimal amount, it may vary depending on the disease to be treated and the presence or absence of side effects, and the optimal dosage may be determined using routine experiments. The pharmaceutical composition may be administered by periodic injections of a bolus of the composition, or may be administered by continuous intravenous, subcutaneous, or intraperitoneal administration from an external (e.g., an intravenous bag) or internal (e.g., a bioerodible implant) reservoir.

Suitable routes include oral, parenteral (including subcutaneous, intramuscular, intravenous, intraarterial, inhalational, intradermal, intrathecal, epidural, and infusion techniques), transdermal, rectal, intranasal, topical (including buccal and sublingual), vaginal, intraperitoneal, intrapulmonary, and intranasal administration. Topical administration may involve the use of transdermal administration such as transdermal patches or iontophoresis devices.

The preferred route may vary depending on, for example, the condition of the recipient. Where the pharmaceutical composition is administered orally, it may be formulated as a pill, capsule, tablet, etc. with a pharmaceutically acceptable carrier, lubricant, or excipient. Where the pharmaceutical composition is administered parenterally, it may be formulated with a pharmaceutically acceptable parenteral vehicle or diluent, and in a unit dosage injectable form.

The pharmaceutical composition may further comprise a pharmaceutically acceptable carrier. The carrier may be, but is not limited to, at least one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, which are commonly used in the formulation of drugs. In addition, the pharmaceutical composition may further comprise at least one selected from the group consisting of diluents, excipients, lubricants, wetting agents, sweeteners, flavoring agents, emulsifiers, suspending agents, and preservatives, which are commonly used in the preparation of pharmaceutical compositions.

The effective amount of the pharmaceutical composition may be administered orally or parenterally. For parenteral administration, the pharmaceutical composition may be administered by intravenous injection, subcutaneous injection, intramuscular injection, intraperitoneal injection, intradermal administration, topical administration, intranasal administration, intrapulmonary administration, and rectal administration. Since proteins or peptides are digested when administered orally, oral compositions may be formulated to coat the active agent or protect the active agent from degradation in the stomach. Additionally, the composition may be administered by any device that allows the active agent to be transferred to a target cell.

The present invention also relates to a method for preventing or treating Fabry disease (FD), comprising administering to a subject an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor. The subject may be a mammal, preferably a human.

"Treating" or "treatment" refers to any indicia of success in the treatment or amelioration of an injury, disease, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; or improving a patient's physical or mental well-being. The treatment or amelioration of symptoms can be based on objective or subjective parameters; including the results of a physical examination, neuropsychiatric exams, and/or a psychiatric evaluation.

An "effective amount" is generally an amount sufficient to reduce the severity and/or frequency of symptoms, eliminate the symptoms and/or underlying cause, prevent the occurrence of symptoms and/or their underlying cause, or improve or remediate the damage that results from or is associated with the disease state.

The effective amount is a therapeutically effective amount or a prophylactically effective amount. A "therapeutically effective amount" is an amount sufficient to remedy a disease state or symptoms, particularly a state or symptoms associated with the disease state, or otherwise prevent, hinder, retard or reverse the progression of the disease state or any other undesirable symptom associated with the disease in any way whatsoever. A "prophylactically effective amount" is an amount of a pharmaceutical composition that, when administered to a subject, has the intended prophylactic effect, e.g., preventing or delaying the onset of a disease state, or reducing the likelihood of onset (or recurrence) of a disease state or associated symptoms.

The present invention also relates to a pharmaceutical composition for preventing or treating Fabry disease (FD), comprising, as an active ingredient, an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, and being for use in combination with agalsidase-β (agal).

The TGF-β receptor activity inhibitor may be SB431542 or D-4476 comprising the following structure or a pharmaceutically acceptable salt thereof: 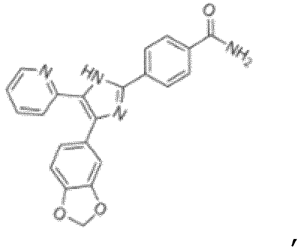 or

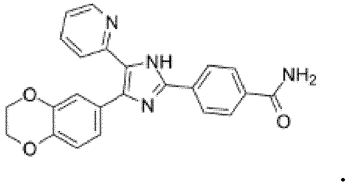

The PPARγ antagonist may be T0070907 comprising the following structure or a pharmaceutically acceptable salt thereof:

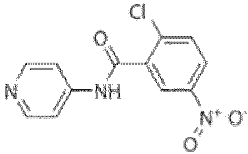

The calcium channel blocker may be lomerizine comprising the following structure or a pharmaceutically acceptable salt thereof:

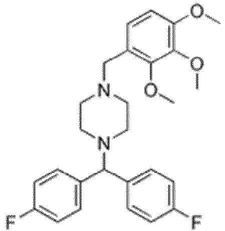

The COX inhibitor may be tolfenamic acid comprising the following structure or a pharmaceutically acceptable salt thereof:

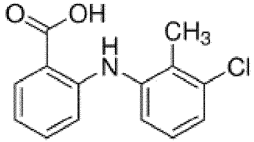

The piperazine-based compound may be eprazinone or a pharmaceutically acceptable salt thereof:

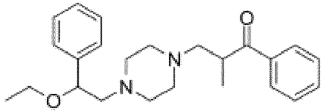

The Rho kinase inhibitor may be fasudil comprising the following structure or a pharmaceutically acceptable salt thereof:

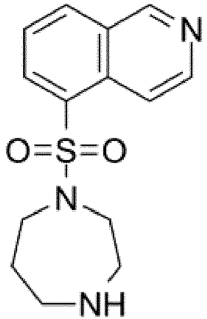

The ROCK inhibitor may comprise at least one selected from the group consisting of Y27632, GSK429286A, Y39983, belumosudil, netarsudil mesylate, ripasudil hydrochloride hydrate, AR-13503 (main netarsudil metabolite), AMA-0076 (sovesudil), AT-13148, and VX-210, or a pharmaceutically acceptable salt thereof.

Preferably, the agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, and agalsidase-β (agal) have complementary activities, and thus they do not adversely affect each other.

The composition according to the present invention and agalsidase-β (agal) may be (1) co-formulated and administered or delivered simultaneously in a combined formulation; or (2) administered or delivered simultaneously or sequentially as separate formulations.

In the case of the combined formulation, the agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, and agalsidase-β (agal) may be present in the same composition. The formulation may be, for example, a dried powder composition, a solution, or a suspension, without being limited thereto.

The agent selected from the group consisting of a TGF-β (transforming growth factor-β) receptor activity inhibitor, a PPARγ (peroxisome proliferator-activated receptor) antagonist, calcium channel blocker, a COX (cyclooxygenase) inhibitor, a piperazine-based compound, a Rho kinase inhibitor and a ROCK (Rho-associated protein kinase) inhibitor, and agalsidase-beta may be administered simultaneously or sequentially.

The agent selected from the group consisting of a TGF-β (transforming growth factor-β) receptor activity inhibitor, a PPARγ (peroxisome proliferator-activated receptor) antagonist, calcium channel blocker, a COX (cyclooxygenase) inhibitor, a piperazine-based compound, a Rho kinase inhibitor and a ROCK (Rho-associated protein kinase) inhibitor, and agalsidase-beta may generally be separated from each other and administered simultaneously or sequentially. Sequential administration thereof may be administration as two or more doses. If they are administered sequentially, one of the agents selected from the group consisting of a TGF-β (transforming growth factor-β) receptor activator inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, and agalsidase-beta may be administered, followed by the other. Alternatively, the agent selected from the group consisting of a TGF-β (transforming growth factor-β) receptor activator inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, and agalsidase-beta may be administered alternately with an interval between the two administrations.

The dosage of agalsidase-beta may be an amount commonly used in the art, and the dosage may be reduced due to its use in combination with the agent selected from the group consisting of a TGF-β (transforming growth factor-β) receptor activator inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor.

### Examples

Hereinafter, the present invention will be described in more detail by way of examples. These examples are merely intended to illustrate the present invention, and it will be apparent to those skilled in the art that the scope of the present invention is not to be construed as being limited by these examples.

### Example 1. Screening of Compounds from Clinical Chemical Library Using FD-VECs

FD-iPSC lines that were generated from fibroblasts of three FD patients with a GLA variant were used. A wild-type (WT) iPSC line derived from foreskin fibroblasts and a FD1 isogenic iPSC line were used as controls. Human iPSCs (hiPSCs) were maintained in mTeSR1 medium (STEMCELL Technologies, Vancouver, Canada) on culture plates precoated with Corning Matrigel^{®} hESC-Qualified Matrix. The cells were maintained at 37°C in a 5% CO₂ incubator. After 7 days of culture, hiPSC colonies were split at a 1:50 ratio using accutase solution (eBioscience, San Diego, CA, USA) and incubated under the same culture conditions for expansion.

Human iPSCs were differentiated into VECs as described previously, with further optimization. Briefly, hiPSCs were transferred to dishes coated with Matrigel^{®} (BD Biosciences, Franklin Lakes, NJ, USA) and cultured for 2 days in mTESR1 medium (STEMCELL Technologies) at 37°C and 5% CO₂. Subsequently, the cells were cultured for a further 2 days in VEC medium (RPMI (HyClone, Logan, MI, USA), 1% B27 (Invitrogen, Carlsbad, CA, USA), and penicillin/streptomycin (Invitrogen)) supplemented with 50 ng/mL Activin A (PeproTech, Rocky Hill, NJ, USA) and 20 ng/mL BMP4 (PeproTech), and 3 µM CHIR990921 (Sigma, St. Louis, MO) at 37°C and 5% CO₂. Next, the cells were cultured for 3 days in VEC medium containing 50 ng/mL VEGF-A (PeproTech) and 50 ng/mL bFGF, at 37°C and 5% CO₂. To differentiate the vascular progenitors into VECs, CD31⁺ cells were sorted from the differentiated cells by MACS using CD31⁺ Dynabeads (Thermo Fisher Scientific, Waltham, MA, USA). The CD31⁺ cells were transferred to gelatin-coated plates and incubated for 3 days in EGM-2 medium (Lonza, Basel, Switzerland) supplemented with 100 ng/mL VEGF-A and 50 ng/mL bFGF, at 37°C and 5% CO₂. The culture medium was changed daily. Finally, the vascular progenitors were cultured in EGM-2 medium without growth factors for 4 days with daily medium change.

To screen for clinical compounds that could rescue FD-VEC dysfunction, VECs were seeded onto gelatin-coated 96-well plates (SPL Life Sciences, Pocheon, Korea) at a density of 3 × 10³ cells/cm² and maintained in EGM-2 medium without growth factors for 24 hours. FD-VECs were treated with each compound (5 µM) for 24 hours, and chemical compounds that did not affect cell viability or morphology were selected. Subsequently, it was examined whether FD-VECs treated with each selected compound could form tube-like structures on Matrigel^{®}-coated plates. The tube-like structure forming assay was repeated at least three times using different concentrations of each selected compound (0.5, 1, and 5 µM).

FIG. 1A shows a schematic view of the process for screening clinical compounds. FD-VECs were obtained by differentiation from FD-iPSCs as previously described (Do et al., 2020). Briefly, a cell population including CD31⁺ cells was obtained from FD-iPSCs, sorted using CD31⁺ magnetic beads, and matured to FD-VECs for 7 days *in vitro.* A library of 2,107 FDA-approved, preclinical and clinical compounds was used in the screening assay. In the first screening, 451 chemical compounds were selected on the basis of a lack of cytotoxicity and/or no aberrant morphological changes of FD-VECs. In the second screening, 15 compounds were selected based on their ability to promote the formation of tube-like structures by FD-VECs. The tube lengths of FD-VECs incubated with these 15 compounds (treatment concentration: 5 µM) were at least 1.5-fold higher than those of control FD-VECs treated with DMSO and were also higher than those of FD-VECs treated with the TGF-β inhibitor (SB431542) (FIG. 1B). Next, the tube-like structure forming assay using various concentrations (0.5, 1, and 5 µM) of the 15 compounds was repeated, and it was found that six compounds induced relatively higher tube formation ability than that induced by the DMSO control (FIG. 1C). The selected compounds included two preclinical compounds (a TGF-β receptor inhibitor (D-4476) and a PPARγ antagonist (T0070907)), and four clinical compounds (a calcium channel blocker (lomerizine), a COX inhibitor (tolfenamic acid), a member of the class of piperazines (eprazinone)), and a RhoA/Rho kinase (ROCK) inhibitor (fasudil). FD-VECs treated with the six selected compounds were able to form tube-like structures, respectively. In addition, Western blot analysis revealed that each compound downregulated the levels of p-SMAD2 and TSP1 in FD-VECs, and significantly increased the levels of the angiogenic factors KDR and eNOS (FIG. 1D). Thus, the present inventors established a novel phenotypic screening platform for drug repurposing that involves examination of the *in vitro* functionality of FD-VECs.

### Example 2. Fasudil improves the defective tube formation ability of FD-VECs

Among the six compounds selected via the screening analysis, fasudil was chosen for further analysis because it was the most effective at promoting the tube-like structure formation of FD-VECs.

FD-VECs were dissociated after treatment with Accutase (eBioscience) at 37°C for 5 min. Dissociated cells (1 × 10⁴) were transferred to Matrigel^{®} matrix (BD Biosciences) in EGM-2 medium supplemented with 100 ng/mL VEGF-A, and then incubated at 37°C and 5% CO₂ for 24 hours. Images of the vascular tube-like structures were taken using an inverted microscope (Olympus, Tokyo, Japan). Total tube lengths were measured using ImageJ software, which was freely provided by the National Institute of Mental Health (NIMH; Bethesda, MD, USA), and its Angiogenesis Analyzer plugin (Carpentier, 2012).

On the basis of the formed tube-like structure length, it was determined that the optimal concentration and half maximal effective concentration (EC₅₀) of fasudil were 5 µM and 0.4183 µM, respectively (FIG. 2A). Therefore, a 5 µM concentration was used in subsequent experiments. Treatment with 5 µM fasudil significantly increased the tube length of FD-VECs compared to the untreated group, and the tube length of the fasudil-treated FD-VECs was comparable to that of gene-corrected FD-VECs (FD(c)-VECs) (FIG. 2B). Fasudil also enhanced the tubular formation ability of VECs derived from FD-iPSC lines with distinct GLA mutations (FIG. 2C). Furthermore, compared to non-treated FD-VECs, both FD(c)-VECs and fasudil-treated FD-VECs had reduced the levels of p-SMAD2 and TSP1 and increased the levels of KDR and eNOS (FIG. 2D). Overall, these findings demonstrate that fasudil is effective in alleviating the defective angiogenesis of FD-VECs *in vitro.*

### Example 3. Fasudil improves the functionality of FD-VECs by downregulating EndMT

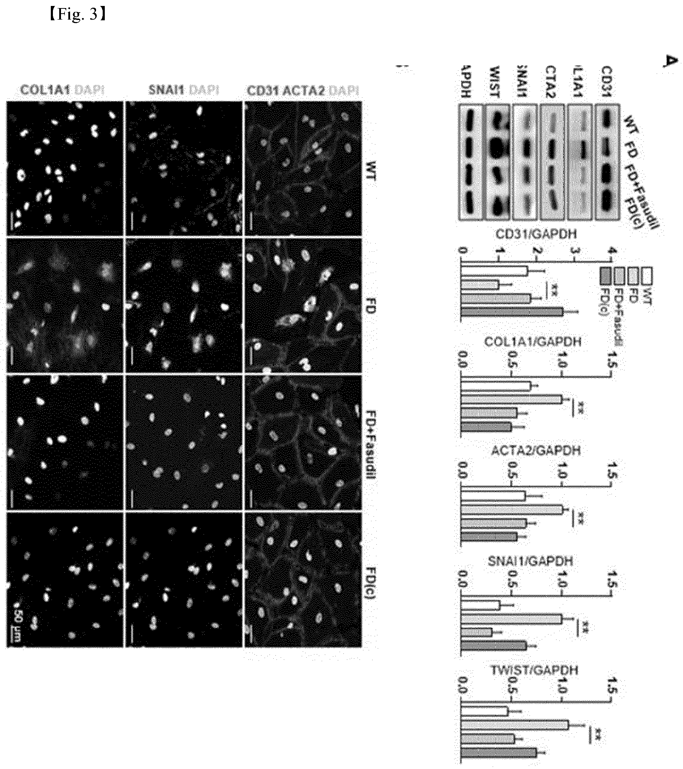

Next, the present inventors examined the mechanism by which fasudil improves the defective angiogenic ability of FD-VECs. Hyperactive SMAD2 signaling induces EndMT in VECs, thereby reducing angiogenic functionality. Given its ability to reduce the expression of p-SMAD2 in FD-VECs, the present inventors examined the effect of fasudil on the EndMT of these cells. Before drug treatment, the present inventors performed bulk RNA sequencing of FD-VECs and FD(c)-VECs to examine the mechanism underlying the defective vasculopathy of FD-VECs. Compared with those in WT-VECs and FD(c)-VECs, the protein level of CD31 was downregulated, and those of mesenchymal-related genes (COL1A1, ACTA2, SNAI1, and TWIST) were upregulated in FD-VECs (FIG. 3A). Treatment with fasudil increased the protein level of CD31 and reduced the levels of mesenchymal-related markers in FD-VECs (FIG. 3A). Immunostaining confirmed that the fluorescence intensities of ACTA2, SNAI1, and COL1A1 were reduced following the treatment of FD-VECs with fasudil (FIG. 3B). Overall, these results demonstrate that the impaired angiogenesis of FD-VECs is associated with increased EndMT caused by hyperactive TGF-β signaling, and fasudil is able to suppress TGF-β-induced EndMT.

### Example 4. Fasudil alleviates impaired metabolic processes in FD-VECs

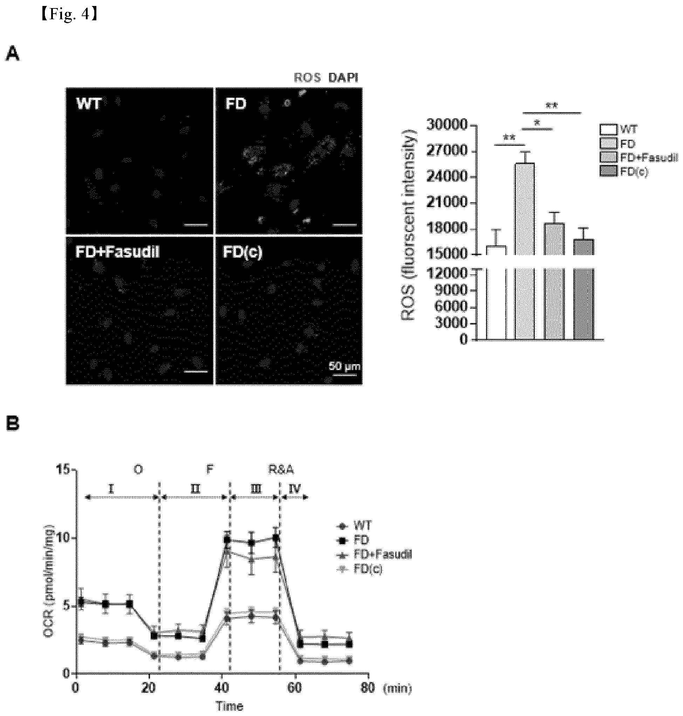

Next, the present inventors examined the mechanism by which fasudil suppresses TGF-β-induced EndMT in FD-VECs. ROS is a key contributor to the vascular damage caused by TGF-β-induced EndMT. As reported previously (Tseng et al., 2017), FD-VECs had a higher level of ROS than WT-VECs (FIG. 4A), but treatment with fasudil reduced the level of ROS to that seen in WT-VECs and FD(c)-VECs. Measurement of the oxygen consumption rate (OCR) in WT-VECs and FD-VECs showed that FD-VECs displayed enhanced basal respiration (I), ATP production (II), maximal respiration (III), and spare capacity (IV). Treatment of FD-VECs with fasudil partially reduced maximal respiration but did not alter basal respiration, ATP production, or spare capacity (FIG. 4B). These results indicate that fasudil partially alleviates impaired metabolic processes by promoting ROS clearance in FD-VECs and regulating mitochondrial oxygen consumption.

### Example 5. Fasudil alleviates FD phenotypes (LVH, anhidrosis, and heat insensitivity) in vivo

Currently available animal models of FD with GLA deficiency do not fully exemplify the vasculopathy of human FD patients. It was found that renal tissue biopsies from FD patients had higher levels of TSP1 than those from healthy donors. Therefore, the present inventors hypothesized that Gla^{-/-} mice expressing human TSP1 (Gla^{-/-}/TSP1^{Tg}; hereinafter referred to as FD mice) might be an effective animal model for studying FD-related vasculopathy. Oral administration of fasudil (10 or 30 mg/kg/day) to FD mice was performed for 6 months, starting from approximately 2 months of age (FIG. 5A). LVH is known to occur in up to half of males and one-third of females in FD patients (Kampmann C, Linhart A, Baehner F, Palecek T, Wiethoff CM, Miebach E, Whybra C, Gal A, Bultas J, Beck M (2008) Onset and progression of the Anderson-Fabry disease related cardiomyopathy. International Journal of Cardiology 130: 367-73). Thus, the present inventors investigated the cardiac functionality in each mouse group via echocardiography. Compared to WT mice, FD mice exhibited a higher left ventricular (LV) mass/body weight (BW) and lower cardiac ejection fraction, fractional shortening of the heart, and cardiac output (FIG. 5B). The LV mass/BW was significantly reduced in FD mice treated with fasudil (10 or 30 mg/kg) for 6 months (FIG. 5B). Furthermore, fasudil administration enhanced the cardiac ejection fraction, fractional shortening of the heart, and cardiac output in FD mice (FIG. 5B). These results indicate that fasudil administration improves the defective cardiac functionality of FD mice.

The present inventors also examined the effect of fasudil on other FD phenotypes, including anhidrosis and heat insensitivity. Compared to WT mice, FD mice showed a reduced number of sweat spots and an increased paw withdrawal latency in a heat tolerance test. However, oral administration of fasudil significantly alleviated anhidrosis and heat insensitivity in FD mice (FIGS. 5C and 5D). Overall, these findings suggest that *in vivo* administration of fasudil alleviates several FD clinical phenotypes in FD mice, including the progression of renal fibrosis and inflammation.

### Example 6. Fasudil alleviates FD phenotypes in vivo

Another major symptom of FD is renal fibrosis. In general, fibrosis occurs via EndMT along with inflammation in VECs of various tissues. Immunohistochemical analysis showed that CD31+ACTA2+ cells were highly distributed in the renal tissues of FD mice compared to those of WT mice, but their amount was reduced in FD mice after fasudil administration (FIG. 6A). In addition, fasudil administration downregulated the expression of COL1A1, another EndMT marker, in the renal tissues of FD mice (FIG. 6B). The expression levels of F4/80 and LCN2, which are inflammation-related proteins, were also increased in the renal tissues of FD mice but were reduced by fasudil administration (FIG. 6C). These findings were confirmed by Western blot analysis of renal tissues from FD mice treated with or without fasudil (FIG. 6D).

### Example 7. Effect of co-treatment with Fasudil and recombinant human agalsidase-β

It was reported that treatment with recombinant human agalsidase-β (agal) had no significant therapeutic effect on improving the impaired tube formation function of FD-VECs (Do HS, Park SW, Im I, Seo D, Yoo HW, Go H, Kim YH, Koh GY, Lee BH, Han YM (2020) Enhanced thrombospondin-1 causes dysfunction of vascular endothelial cells derived from Fabry disease-induced pluripotent stem cells. EBioMedicine 52: 102633). Therefore, the present inventors analyzed whether the co-administration of fasudil and agal could synergistically support the therapeutic effect of fasudil. To this end, co-treatment of FD-VECs with agal and fasudil was performed. FD-VECs were treated with agal every two days and then once with fasudil, and then tube formation was analyzed (FIG. 7A). Surprisingly, the co-treatment rescued the tube formation function of FD-VECs more effectively than treatment with fasudil alone (FIG. 7B). In addition, the co-treatment efficiently reduced the levels of TSP1 and p-SMAD2 in FD-VECs and increased the expression of angiogenic factors (KDR and eNOS) compared to each treatment alone (FIG. 7C). The co-treatment effectively reduced ROS generation in FD-VECs more effectively than each treatment alone (FIG. 7D). In addition, agal treatment was effective in reducing basal respiration (I), ATP production (II), maximal respiration (III), and spare capacity (IV) in FD-VECs (FIG. 7E). Surprisingly, co-treatment with fasudil and agal effectively reduced the maximal oxygen consumption rate (OCR) in FD-VECs compared to each treatment alone (FIG. 7E). Therefore, fasudil is a compound that can act as a drug to enhance the therapeutic effect of agalsidase-β in FD-VECs.

### Example 8. Effect of co-treatment with ERT (enzyme replacement therapy) and fasudil

To treat Fabry disease, recombinant enzyme (beta-galactosidase) is injected intravascularly into patients. In this study, the present inventors aimed to examine whether fasudil would have a therapeutic effect even when administered together with enzyme therapy.

### 8-1. Synergistic effect of co-treatment on function of FD vascular endothelial cells

To examine whether vascular endothelial cells derived from Fabry disease-induced pluripotent stem cells form tube-like structures, the cells were treated with the enzyme at two-day intervals and then with fasudil at the end.

The results are shown in FIG. 8. Co-administration of the enzyme and fasudil exhibited a much higher tube formation ability than treatment with the enzyme alone. This means that fasudil has a greater effect on improving the functionality of Fabry disease vascular endothelial cells than the enzyme.

### 8-2. Synergistic effect of co-treatment on enhancing the metabolic function of FD vascular endothelial cells

The co-treatment was performed in the same manner as in Example 8-1. The results are shown in FIG. 9. The co-treatment further increased the expression of angiogenesis-related factors (KDR and eNOS) and relatively further reduced TSP1 expression and TFG-beta activity (FIG. 9A). In addition, the co-treatment reduced ROS production (FIG. 9B) and more effectively reduced maximal respiration OCR (III) (FIG. 9C).

### 8-3. Co-administration in Gla^{-/-} model mice

Based on the results of the *in vitro* experiment, fasudil was orally administered to alpha-galactosidase gene-deficient Fabry disease mice (Gla^{-/-}) at 30 mg/kg daily, and the enzyme (1 mg/kg) was intravenously injected into the tail once a week for 6 months (FIG. 10).

### (1) Echocardiography analysis of mice with Fabry disease after co-administration

The results of echocardiography analysis of mice with Fabry disease after co-administration are shown in FIG. 11.

The experimental group administered the enzyme alone (dark blue) showed a decrease in cardiac function, similar to the Fabry disease group (light blue), whereas the group administered fasudil alone and the group administered fasudil in combination with the enzyme showed an improvement in cardiac function (FIG. 11A), and showed a significant reduction in left ventricular hypertrophy, a typical phenotype of Fabry disease (FIG. 11B). These results indirectly suggest that the current enzyme therapy cannot rescue cardiac function and alleviate left ventricular hypertrophy symptoms.

### (2) Analysis of sweat secretion from mice with Fabry disease after co-administration

The results of analysis of sweat secretion from mice with Fabry disease after co-administration are shown in FIG. 12. The group administered the enzyme alone (dark blue) maintained sweating, like the mice with Fabry disease (light blue), but sweating was reduced in the group administered fasudil alone (light purple) and the group administered fasudil in combination with the enzyme (dark purple). This indicates that the enzyme therapy does not help alleviate sweating symptoms.

### (3) Analysis of peripheral nerves of mice with Fabry disease after co-administration

Many Fabry disease patients suffer from peripheral neuropathic pain, and one way to indirectly assess the pain in laboratory animals is to measure the time it takes for the hind limbs to jump on a hot plate at 55°C. The results are shown in FIG. 13.

Normal mice (gray) jumped on the hot plate with their hind limbs within 20 seconds, while mice with Fabry disease (light blue) lasted more than 30 seconds. The group administered the enzyme alone (dark blue) showed a high latency like the mice with Fabry disease, whereas the group administered fasudil alone and the group co-treated with fasudil and the enzyme showed a slight decrease in latency.

These results indirectly suggest that the enzyme therapy is also ineffective in treating peripheral neuropathic pain in patients with Fabry disease, and fasudil is expected to be helpful in alleviating peripheral neuropathic pain.

### (4) Analysis of kidney tissues of mice with Fabry disease after co-administration

FIG. 14 shows the results of analysis of kidney tissues of mice with Fabry disease after co-administration.

It was confirmed that the expression of a fibrosis-related marker (COL1A1) and inflammation markers (LCN2 and F4/80) was reduced in the kidney tissues of mice with Fabry disease administered with fasudil alone or in combination with the enzyme. This suggests that fasudil can prevent renal failure by inhibiting the promotion of fibrosis, which is the cause of renal failure in patients with Fabry disease.

Because GLA deficiency causes the continuous accumulation of Gb3, an intermediate metabolite, in various tissue cells of patients with Fabry disease, enzyme therapy is necessary to remove Gb3. However, enzyme therapy does not fundamentally treat the symptoms of Fabry disease. Therefore, a new treatment method using co-administration of the enzyme may be able to fundamentally treat patients with Fabry disease.

### Example 9. Lomerizine improves the defective angiogenesis of FD-VECs.

It has been reported that FD-VECs exhibit endothelial dysfunction. Compounds affecting angiogenesis were identified using a cell-based screening platform using FD-VECs, and lomerizine was identified from a library of FDA-approved clinical compounds provided by the Korea Chemical Bank (www.chembank.org) (FIG. 15A). To examine how much lomerizine recues the tube formation ability of FD-VECs, the tube formation ability was observed at various concentrations and EC₅₀ was measured. As a result, it was found that 1 µM is the optimal concentration for maximal effectiveness (FIG. 15B). Lomerizine treatment significantly increased the total tube length of FD-VECs in the tube formation assay (FIG. 15C). In addition, the present inventors found that lomerizine treatment also enhanced the tube formation ability of FD-VECs with distinct GLA mutations (FIG. 15D). Furthermore, lomerizine treatment reduced the expression of p-SMAD2 and TSP1 in FD-VECs and increased the expression of KDR and eNOS in FD-VECs (FIG. 15E). Therefore, lomerizine seems to be an effective therapeutic agent that rescues the functionality of FD-VECs *in vitro.*

### Example 10. Lomerizine improves the mitochondrial dysfunction of FD-VECs

The present inventors explored how lomerizine improves the angiogenic capacity of FD-VECs. Gb3 accumulation has been reported to cause mitochondrial dysfunction, including dysregulation of ROS production (Stepien et al, 2020; Tseng et al., 2017). Any imbalance between ROS production and the antioxidant defense system may lead to dysfunction of vascular endothelial cells. Lomerizine treatment reduced the excess ROS produced in FD-VECs to the same level as that in WT- and FD(c)-VECs (FIG. 16A). It was found that FD-VECs exhibited higher basal respiration (I), ATP production (II), maximal respiration (III), and spare capacity (IV) than WT- and FD(c)-VEC (FIG. 16B). In addition, it was found that FD-VECs exhibited higher basal respiration (I), ATP production (II), maximal respiration (III), and spare capacity (IV) than WT- and FD(c)-VEC (FIG. 16B). Further, in the data, the present inventors observed a slight decrease in maximal respiration when FD-VECs were treated with lomerizine. The above results indicate that lomerizine can effectively regulate ROS production in FD-VECs.

### Example 11. Lomerizine improves FD-VEC function by downregulating EndMT.

Next, the present inventors investigated how lomerizine rescues the dysfunction of vascular endothelial cells of FD-VEC via ROS clearance. As ROS is known to induce EndMT (Piera-Velazquez & Jimenez, 2019), the present inventors found that treatment of FD-VECs with lomerizine reduced the expression of mesenchymal cell-related genes COL1A1, ACTA2, SNAI1, and TWIST compared to that in WT-VEC or FD(c)-VEC (FIG. 17A). In addition, the present inventors found through immunostaining that treatment of FD-VECs with lomerizine reduced the expression of EndMT-related genes (FIG. 17B). Therefore, the above results demonstrate that lomerizine suppresses EndMT in FD-VECs, thereby improving the impaired functionality of vascular endothelial cells.

### Example 12. Oral administration of lomerizine rescues the FD phenotypes of FD mice

Existing FD animal models do not fully recapitulate the vasculopathies in human FD patients (Miller et al, 2019; Ohshima et al, 1999; Taguchi et al, 2013). The previous study conducted by the present inventors showed that Gla^{-/-}/TSP1^{T9} mice effectively recapitulate FD-associated vasculopathy. To this end, FD-mice (Gla^{-/-}/TSP1^{Tg}) were produced by crossing transgenic mice expressing human TSP1 with Gla^{-/-} mice. The produced mice were then orally administered lomerizine (10 or 30 mg/kg/day) for 6 months (FIG. 18A). Using echocardiography, the present inventors found that although FD-mice exhibited a higher ratio of left ventricle (LV) to body weight (BW) than WT mice, lomerizine administration reduced this LV to BW ratio (FIG. 18B). Similarly, although FD-mice exhibited reduced heart function (i.e., cardiac ejection fraction, fractional shortening, and cardiac output) compared to WT mice, long-term lomerizine administration induced significant improvements in heart function. Thus, these results show that lomerizine administration can improve the defective cardiac function of FD mice. In addition, although the number of sweat spots and heat sensitivity were reduced in FD-mice compared to WT mice (FIGS. 18C and 18D), lomerizine administration rescued this phenotype. Overall, these results suggest oral administration of lomerizine alleviates several FD-like symptoms of FD-mice.

### Example 13. Oral administration of lomerizine alleviates fibrosis and inflammation in the renal tissues of FD-mice

Renal fibrosis is another major symptom of FD (Weidemann et al., 2013). Fibrosis and inflammation typically arise when VECs in various tissues (including the kidney) undergo EndMT (Ma et al, 2020). Therefore, the present inventors investigated how oral administration of lomerizine affects tissue fibrosis and inflammation in the renal tissues of FD-mice. To this end, the expression of EndMT-related genes was analyzed in Fabry disease mice (FIG. 19A). The present inventors found significantly more CD31+/ACTA2+cells in the renal tissues of FD-mice compared to WT, but oral administration of lomerizine reduced this increase (FIG. 19B).

Furthermore, it was found that FD-mice exhibited EndMT in the renal microvasculature, but oral administration of lomerizine to FD-mice downregulated the renal expression of the EndMT marker COL1A1 (FIG. 19C) and reduced the increased expression of the inflammation-related proteins F4/80 and LCN (FIG. 19D). When Western blot analysis was performed on the renal tissues from FD mice and FD mice treated with lomerizine, it was shown that lomerizine reduced the protein levels of fibrotic and inflammatory markers (FIG. 19E). Finally, the above results indicate that lomerizine can effectively suppress the progression of renal fibrosis and inflammation in FD mice, and suggest that its use should be explored for human FD patients.

### Example 14. Co-treatment with lomerizine and recombinant human α-galactosidase exhibits effective therapeutic effect

The previous study conducted by the present inventors reported that treatment with recombinant human α-galactosidase (agalsidase-β, agal) had no significant therapeutic effect on improving impaired tube formation function of FD-VECs (Do et al., 2020). Thus, the present inventors analyzed whether co-administration of lomerizine and agal could increase the therapeutic effect of agal. To this end, co-treatment of FD-VECs with agal and lomerizine was performed. FD-VECs were treated with agal every two days and finally treated once with lomerizine, and then the tube formation ability was analyzed (FIG. 20A). Surprisingly, the co-treatment rescued the tube formation function of FD-VECs more effectively than treatment with lomerizine alone (FIG. 20B). In addition, the co-treatment effectively reduced the levels of TSP1 and p-SMAD2 in FD-VECs and increased the expression of angiogenic factors (KDR and eNOS), compared to each treatment alone (FIG. 20C). The co-treatment effectively reduced ROS production in FD-VECs more effectively than each treatment alone (FIG. 20D). In addition, agal treatment was effective in reducing basal respiration (I), ATP production (II), maximal respiration (III), and spare capacity (IV) in FD-VECs (FIG. 20E). Surprisingly, the co-treatment with lomerizine and agal effectively reduced maximal respiration (maximal oxygen consumption rate (OCR)) of FD-VECs compared to each treatment alone (FIG. 20E). Therefore, lomerizine is a compound that can act as a drug to enhance the therapeutic effect of agalsidase-β in FD-VECs.

### Example 15. ROCK inhibitors

### [Types of ROCK inhibitors used in the experiment]

First experiment: To confirm whether decreased tube formation was rescued when vascular endothelial cells derived from Fabry disease-induced pluripotent stem cells were treated with 4 types of ROCK inhibitors (Y27632, GSK429286A, Y39983, and belumosudil) at a concentration of 5 µM, a tube formation experiment was conducted (Table 1).

**[Table 1] ROCK inhibitors used in the first experiment**

| **Drug Name** | **IC50** |
|---|---|
| Y27632 | Rock1 (140nM), Rack2 (300nM) |
| GSK429286A | Rock1 (14nM), Rock2 (63nM) |
| Y39983 | Rock (3.6nM) |
| Belumosudil | Rock2 (41-60nM) |

Second experiment: To confirm whether decreased tube formation in vascular endothelial cells derived from Fabry disease-induced pluripotent stem cells was rescued by treatment with 7 types of ROCK inhibitors (netarsudil, ripasudil, sovesudil, AT13148, AR13503, VX-210, and latanoprost), a tube formation experiment was conducted (Table 2).

**[Table 2] Types of ROCK inhibitors used in the second experiment**

| **Drug Name** | **IC50** |
|---|---|
| Netarsudil mesylate | Rock1 (1-2nM), Rock2 (1-2nM) |
| Ripasudil hydrochloride hydrate | Rock1 (51nM) Rock2 (19nM) |
| AR-13503 (Main Netarsudil Metabolite) | Rock1 (N.A) Rock2 (4.2nM) |
| AMA-0076 (Sovesudil) | Rock1 (3.7nM) Rock2 (23nM) |
| AT-13148 | Rock1 (6nM) Rock2 (4nM) |
| VX-210 | NA |

### Example 16. Efficacy evaluation

Matrigel was applied to a 96-well plate which was then stored in a 5% CO₂ incubator a 37°C for one day. The next day, each ROCK inhibitor was added to each well at various concentrations (5 µM, 1 µM, 0.5 µM, 0.1 µM, 0.05 µM, and 0.01 µM) together with 100 µg/ml VEGF in EGM-2 media.

10,000 vascular endothelial cells per well were applied onto the solidified Matrigel, and after one day, each well was photographed under a microscope. Then, the total tube length was analyzed using Image J to compare the increase and decrease in tube formation.

As a result of the first experiment, referring to FIGS. 21 to 25, when the Fabry disease vascular endothelial cells were treated with each of Y27632, GSK429286A, Y39983, and belumosudil at a concentration of 5 µM, each of the compounds was observed to be effective in improving the impaired tube formation ability of the Fabry disease vascular endothelial cells.

As a result of the second experiment, which was conducted additionally, as shown in FIGS. 26 to 32, it was confirmed that the tube formation ability of vascular endothelial cells derived from Fabry disease was lower than that of normal vascular endothelial cells, and that the Fabry disease-derived vascular endothelial cell group treated with SB431542 (SMAD2 signaling inhibitor) as a control had an increased tube formation ability. In addition, it was confirmed that netarsudil, ripasudil, sovesudil, AT13148, AR13503, and VX-210 used in the second experiment all increased the tube formation ability of Fabry disease vascular endothelial cells at a concentration of 5 µM.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A pharmaceutical composition for preventing or treating Fabry disease (FD), comprising, as an active ingredient, an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor.

2. The pharmaceutical composition of claim 1, wherein the TGF-β receptor activity inhibitor is SB431542 or D-4476 comprising the following structure or a pharmaceutically acceptable salt thereof: 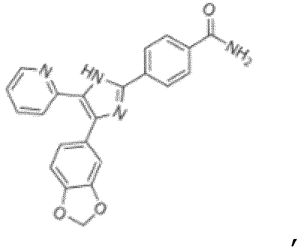 or

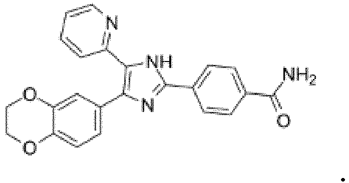

3. The pharmaceutical composition of claim 1, wherein the PPARγ antagonist is T0070907 comprising the following structure or a pharmaceutically acceptable salt thereof:

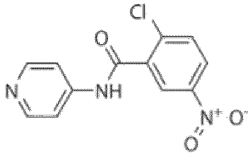

4. The pharmaceutical composition of claim 1, wherein the calcium channel blocker is lomerizine comprising the following structure or a pharmaceutically acceptable salt thereof:

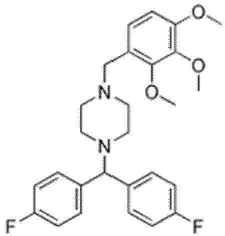

5. The pharmaceutical composition of claim 1, wherein the COX inhibitor is tolfenamic acid comprising the following structure or a pharmaceutically acceptable salt thereof:

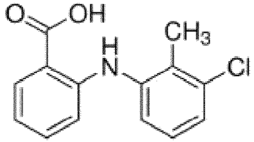

6. The pharmaceutical composition of claim 1, wherein the piperazine-based compound is eprazinone or a pharmaceutically acceptable salt thereof:

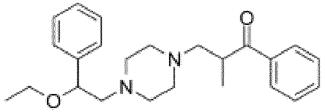

7. The pharmaceutical composition of claim 1, wherein the Rho kinase inhibitor is fasudil comprising the following structure or a pharmaceutically acceptable salt thereof:

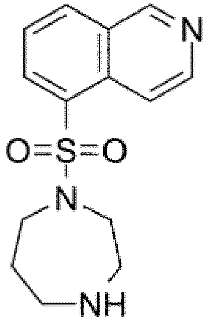

8. The pharmaceutical composition of claim 1, wherein the ROCK inhibitor comprises at least one selected from the group consisting of Y27632, GSK429286A, Y39983, belumosudil, netarsudil mesylate, ripasudil hydrochloride hydrate, AR-13503 (main netarsudil metabolite), AMA-0076 (sovesudil), AT-13148, and VX-210, or a pharmaceutically acceptable salt thereof.

9. The pharmaceutical composition of claim 1, exhibiting the following characteristic:
reducing p-SMAD2 or TSP1 protein;
increasing the level of angiogenic factor KDR or eNOS;
reducing reactive oxidative stress (ROS) and maximal respiration;
alleviating a Fabry disease phenotype selected from the group consisting of left ventricular hypertrophy (LVH), renal fibrosis, anhidrosis, and heat intolerance; or
improving impaired tube formation ability of vascular endothelial cells.

10. A pharmaceutical composition for preventing or treating Fabry disease (FD), comprising, as an active ingredient, an agent selected from the group consisting of a TGF-β (transforming growth factor-β) receptor activity inhibitor, a PPARγ (peroxisome proliferator-activated receptor) antagonist, a calcium channel blocker, a COX (cyclooxygenase) inhibitor, a piperazine-based compound, a Rho kinase inhibitor, and a ROCK (Rho-associated protein kinase) inhibitor, and being for use in combination with agalsidase-β (agal).

11. The pharmaceutical composition of claim 10, wherein the TGF-β receptor activity inhibitor is SB431542 or D-4476 comprising the following structure or a pharmaceutically acceptable salt thereof: 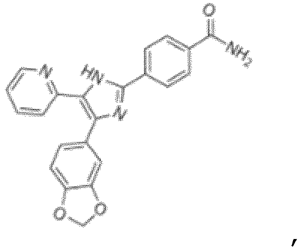 or

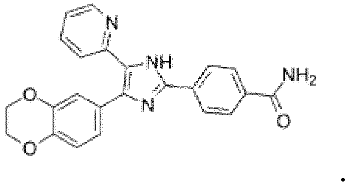

12. The pharmaceutical composition of claim 10, wherein the PPARγ antagonist is T0070907 comprising the following structure or a pharmaceutically acceptable salt thereof:

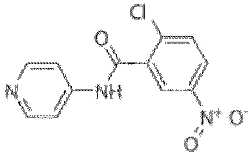

13. The pharmaceutical composition of claim 10, wherein the calcium channel blocker is lomerizine comprising the following structure or a pharmaceutically acceptable salt thereof:

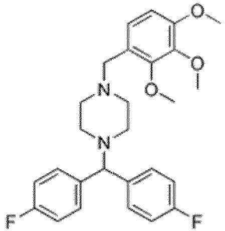

14. The pharmaceutical composition of claim 10, wherein the COX inhibitor is tolfenamic acid comprising the following structure or a pharmaceutically acceptable salt thereof:

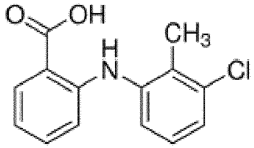

15. The pharmaceutical composition of claim 10, wherein the piperazine-based compound is eprazinone or a pharmaceutically acceptable salt thereof:

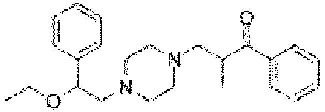

16. The pharmaceutical composition of claim 10, wherein the Rho kinase inhibitor is fasudil comprising the following structure or a pharmaceutically acceptable salt thereof:

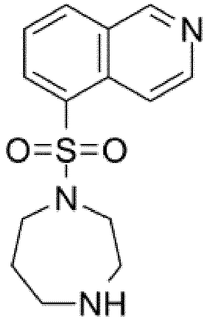

17. The pharmaceutical composition of claim 10, wherein the ROCK inhibitor comprises at least one selected from the group consisting of Y27632, GSK429286A, Y39983, belumosudil, netarsudil mesylate, ripasudil hydrochloride hydrate, AR-13503 (main netarsudil metabolite), AMA-0076 (sovesudil), AT-13148, and VX-210, or a pharmaceutically acceptable salt thereof.

18. The pharmaceutical composition of claim 10, wherein the agent selected from the group consisting of the TGF-β (transforming growth factor-β) receptor activity inhibitor, PPARγ (peroxisome proliferator-activated receptor) antagonist, calcium channel blocker, COX (cyclooxygenase) inhibitor, piperazine-based compound, Rho kinase inhibitor and ROCK (Rho-associated protein kinase) inhibitor, and the agalsidase-beta are comprised in a combination formulation.

19. The pharmaceutical composition of claim 10, wherein the agent selected from the group consisting of the TGF-β (transforming growth factor-β) receptor activity inhibitor, PPARγ (peroxisome proliferator-activated receptor) antagonist, calcium channel blocker, COX (cyclooxygenase) inhibitor, piperazine-based compound, Rho kinase inhibitor and ROCK (Rho-associated protein kinase) inhibitor, and the agalsidase-beta are administered simultaneously or sequentially.
